# EUROPEAN PATENT APPLICATION

(11) **EP 1 728 784 A1**
(43) Date of publication of application: **06.12.2006**
(21) Application number: 05710602.3
(22) Date of filing: 17.02.2005
(51) Int. Cl.: C07C 279/22

(54) **FLUORENE DERIVATIVE**

(30) Priority: 20.02.2004 JP 2004044122
(71) Applicant: Astellas Pharma Inc., Tokyo 103-8411 (JP)
(72) Inventor: YAMADA, Hiroyoshi c/o Astellas Pharma Inc., Chuo-ku, Tokyo 1038411 (JP); ITAHANA, Hirotsune c/o Astellas Pharma Inc., Tokyo 103-8411 (JP); MORITOMO, Ayako c/o Astellas Pharma Inc., Chuo-ku, Tokyo 1038411 (JP); MATSUZAWA, Takaho, 3-11, Nihonbashi-Honcho 2-chome Chuo-ku, (JP); HARADA, Koichiro, Oda-gun, Okayama 7141401 (JP); FUJIYASU, Jiro c/o Astellas Pharma Inc., Chuo-ku, Tokyo 1038411 (JP); KOGA, Yuji c/o Astellas Pharma Inc., Chuo-ku, Tokyo 1038411 (JP); OKU, Makoto c/o Astellas Pharma Inc., Chuo-ku, Tokyo 1038411 (JP); OKAZAKI, Toshio c/o Astellas Pharma Inc., Chuo-ku, Tokyo 1038411 (JP); AKUZAWA, Shinobu c/o Astellas Pharma Inc., Chuo-ku, Tokyo 1038411 (JP); WATANABE, Toshihiro c/o Astellas Pharma Inc., Chuo-ku, Tokyo 1038411 (JP)
(74) Representative: HOFFMANN EITLE
(86) International application number: PCT/JP2005/002950
(87) International publication number: WO 2005/080322

(57) **Abstract**

This invention relates to a novel fluorene derivative having a characteristic structure in which guanidino group or the like functional group is linked to the fluorene structure via carbonyl group, or a salt thereof.

The compound of the invention has an advantage in that it has high affinity for serotonin receptor subtypes, particularly for 5-HT_{2B} receptor and 5-HT₇ receptor, and shows excellent pharmacological effects in comparison with the conventional compounds which have only one of the antagonistic activities of 5-HT_{2B} receptor and 5-HT₇ receptor, this is useful as a prophylactic antimigraine agent having high safety and excellent effect.

## Description

### Technical Field

This invention relates to fluorene derivatives useful as medicines, particularly as prophylactic antimigraine agents.

### Background of the Invention

Migraine is a periodically occurring pulsating headache which is a disease in which a strong pain occurs in one side or both sides of the head and is continued for several hours to about 3 days. It is suggested that the morbid state of this migraine advances by the following onset mechanism. Firstly, dura mater blood vessel once contracts by the action of 5-HT (serotonin) or the like neurotransmitter and then expands again, and in this case, inflammation advances by releasing CGRP or the like vasoactive peptide and plasma protein, thus resulting in the onset of headache.

The medicines targeting at migraine are divided into agents for prevention and agents for treatment. The former aims at reducing attack frequency by continuously administering them preventively before onset of the disease, and the latter aims at suppressing the pain by taking them after expression of the attack. Particularly as the preventive agents, Ca antagonists (e.g., lomerizine, flunarizine and the like), serotonin antagonists (e.g., pizotifen, methysergide and the like), β-adrenergic blocking drugs (e.g., propranolol and the like) and the like are clinically used in some country, but many side effects have been reported on all of them, and sufficient clinical effects have not been obtained yet.

Regarding the pizotifen as a serotonin antagonist among the aforementioned preventive agents, its efficacy is high in comparison with other agents, but there is a problem in that fatigue, sleepiness, giddiness, weight gain and the like side effects are found by its effective dose (J. Neurol. (1991) 238, S 45 - S 52). It is known that said compound has the affinity for all of the 5-HT receptor subtypes and also has high affinity for α₁, M₁, D₂ and the like various receptors.

5-HT is a monoamine neurotransmitter and expresses various physiological actions via a 5-HT receptor. The 5-HT receptor is classified into 7 families of from 5-HT₁ to 5-HT₇, and particularly in the case of 5-HT₂ receptor, 3 kinds of subtypes 5-HT_{2A}, 5-HT_{2B} and 5-HT_{2C} are known (Pharmacol. Rev. (1994) 46, 157 - 203). It has been suggested that this 5-HT is deeply concerned in the onset of migraine (Headache (1994) 34, 408 - 417). In addition, it has been reported that an agent having 5-HT receptor antagonism is effective in preventing migraine (Prog. Drug. Res. (1998) 51, 219 - 244) .

In recent years, pharmacological studies on 5-HT receptor subtypes have been conducted. For example, it has been reported that a 5-HT_{2B} receptor antagonist inhibits leakage of protein into outside of guinea pig mCPP induced dural blood vessel (Cephalalgia (2003) 23, 117 - 123), and that the 5-HT_{2B} receptor localizing on vascular smooth muscle causes NO release, and the NO accelerates release of CGRP, substance P and the like nervous peptides from trigeminal nerve (J. Biol. Chem. (2000) 275, 9324 - 9331, Circ. Res. (1992) 70, 1313 - 1319). In addition, a result which suggests the effect to prevent migraine has been obtained by an animal model test using a compound (RS-127445) having selective binding affinity for the 5-HT_{2B} receptor (Clustar Headache and Related Conditions, vol. 9, edited by D. W. Bonhaus (England), Oxford University Press (1999), 278 - 286) .

In addition, there are reports stating that 5-HT, receptor is distributed in the trigeminal nerve (Neurosci. Lett. (2001) 302, 9 - 12) and concerned in the vasodilation by 5-HT in the cerebrovascular smooth muscle, or concerned in the extra-dural blood vessel protein leakage acceleration action (Regional Anesth. (1996) 21, 219 - 225).

In another report, there is a description stating that 5-HT_{1D}, 5-HT_{2B} and 5-HT₇ receptors are present in dural blood vessel (FEBS Lett. (1995) 370, 215 - 221).

Thus, pharmacological relationship between 5-HT receptor subtypes and onset of migraine has been studied, but the subtype as an effective target for the prevention of migraine has not been clearly specified yet.

On the other hand, 5-HT_{2B} receptor antagonistic compounds having selective binding affinity for 5-HT_{2B} receptor (to be referred to as 5-HT_{2B} selective antagonistic compounds hereinafter) have been known, such as RS-127445 (British Journal of Pharmacology (1999) 127, 1075 - 1082), LY-266097 (J. Serotonin Res. (1996) 3, 131), SB-200646 (J. Med. Chem. (1993) 36, 1104), SB-204741 (J. Med. Chem. (1995) 38, 855), SB-206553 (J. Med. chem. (1996) 39, 2773), SB-221284 (9th RSC-SCI Medicinal Chemistry Symposium (1997) P1 (Poster), 7 Sep), EGIS-7625 (Cardiovascular Drugs and Therapy (2003) 17, 427 - 434), a 4-(thio or selenoxanthen-9-ylidene)piperidine or acridine derivative (US 2003166672), a 2-oxazoleamine derivative (WO 2003068226), a 2-thiazoleamine derivative (WO 2003068227) and the like.

In addition, 5-HT₇ receptor antagonistic compounds having selective binding affinity for 5-HT₇ receptor (to be referred to as 5-HT₇ selective antagonistic compounds hereinafter) have been known, such as DR-4004 (J. Med. Chem. (1999) 42, 533), SB-269970 (J. Med. Chem. (2000) 43, 342 - 345), SB-691673 (Bioorg. Med. Chem. (2003) 13, 1055 - 1058), an aminotriazole derivative (Bioorg. Med. Chem. (2004) 14, 4245 - 4248), an aminotetralin derivative (J. Med. Chem. (2004) 47, 3927 - 3930), an aminochroman derivative (J. Med. i. (2004) 47, 3927 - 3930), a 11-phenyapomorphine derivative (J. Med. Chem. (2001) 44, 1337 - 1340), and the like.

There are no substances having the fluorene structure in the aforementioned 5-HT_{2B} selective antagonistic compounds and 5-HT₇ selective antagonistic compounds.

In this connection, there is a reference describing that a 5-HT_{2B} selective antagonistic compound is effective for pulmonary hypertension (Nature Medicine (2002) 8, 1129 - 1135). In addition, facts that certain 5-HT_{2B} selective antagonistic compounds have anti-depression and anti-anxiety actions (American Chemical Society, abstracts of 228th national meeting, part XIII, 2004, pp. 22 - 26) and are effective for sleeplessness (American Society of Neuroscience, abstracts, 1998, vol. 24, part 1, no. 466.9) have been reported at respective scientific meetings.

There is a report suggesting that an acylguanidine derivative represented by the following general formula has AMPA antagonism and is effective in treating various central diseases including migraine (Patent Reference 1). However, said derivative has a cyclic group as R³, and there is no disclosure in said application about illustrative compounds having the fluorene structure as R. 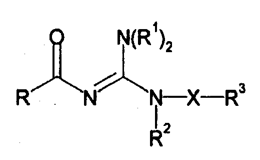 (In the formula, R is a cycloalkyl, an aryl, a monocyclic to tricyclic heteroaryl or the like, R¹ and R² are each independently -H, an alkyl, an alkenyl or the like, X is a bond, an alkene, alkenylene or the like, and R³ is a cycloalkyl, an aryl, an alkyl-aryl or the like. See said official gazette for details.)

In addition, it has been reported that a tricyclic compound represented by the following general formula is possessed of an NO synthase inhibitory activity (Patent Reference 2). Said compound is possessed of a specified cyclic group as A represented by the following formula. 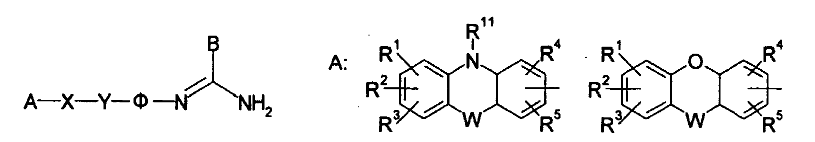 (In the formula, Φ is a bond or phenylene group, B is-CH₂-NO₂, an alkyl group, an aryl group or NR¹³R¹⁴ (R¹³ and R¹⁴ are each independently hydrogen atom, an alkyl group, a cyano group or the like), X is a bond, -O-, -S-, CO- or the like, Y is a bond, -(CH₂)ₘ- or the like, W is not present or a bond, S atom or NR¹⁵, R¹ to R⁵ are hydrogen, a halogen or the like. See said official gazette for details.)

As described in the foregoing, great concern has been directed toward a prophylactic antimigraine agent having excellent effect to prevent migraine and reduced side effects found in the existing prophylactic antimigraine agents.
(Patent Reference 1) International Publication No. 99/20599 pamphlet
(Patent Reference 2) International Publication No. 00/17191 pamphlet

As described in the foregoing, great concern has been directed toward a prophylactic antimigraine agent having excellent effect to prevent migraine and in which the side effects found in the existing prophylactic antimigraine agents are reduced.

### Disclosure of the Invention

As a result of carrying out intensive examinations on a serotonin antagonist having a migraine preventing effect, the present inventors have accomplished the invention by finding that a novel fluorene derivative having a characteristic structure in which a functional group (e.g., guanidino group or the like) is linked to the fluorene structure via carbonyl group or the like, as shown in the following general formula (I), is possessed of excellent preventive effect on migraine. The compound of the invention has high affinity for particularly 5-HT_{2B} receptor and 5-HT₇ receptor among the serotonin receptor subtypes, and has significant effect in comparison with the conventional compounds having only one of the antagonistic activity of 5-HT_{2B} receptor or 5-HT₇ receptor. In addition, since the compound of the invention is possessed of weak antagonism of other than 5-HT_{2B} receptor and 5-HT₇ receptor, side effects caused by these receptors are reduced.

That is, the invention relates to the following compounds.
[1] A fluorene derivative represented by the following general formula (I) or a pharmaceutically acceptable salt thereof 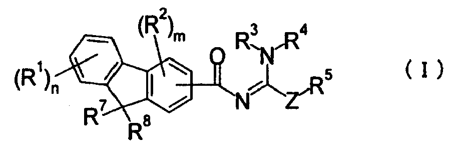 (symbols in the formula represent the following meanings
   R¹ and R²: the same or different from each other and each represents -R⁰, a lower alkenyl, a lower alkynyl, a halogen,
   -OH, -O-R⁰, -O-CO-R⁰, -NH₂, -NR⁶-R⁰, -CN, -NO₂,
   -CHO, -CONH₂, -CO-NR⁶-R⁰, -CO₂H, -CO₂-R⁰, -CO-R⁰, -NR⁶-CO-R⁰,
   -NR⁶-CO₂-R⁰, -O-CO-NR⁶-R⁰, -SH, -S(O)ₚ-R⁰, -S(O)₂-NH₂,
   -S(O)₂-NR⁶-R⁰, -NR⁶-S (O)₂-R⁰, -R⁰⁰-O-CO-R⁰, -R⁰⁰-NR⁶-R⁰, -R⁰⁰ -CN,
   -R⁰⁰-CONH₂, -R⁰⁰-CO-NR⁶-R⁰, -R⁰⁰-CO₂H, -R⁰⁰-CO₂-R⁰, -R⁰⁰-CO-R⁰,
   -R⁰⁰-NR⁶-CO-R⁰, -R⁰⁰-NR⁶*-*CO₂-R⁰, -R⁰⁰-O-CO-NR⁶-R⁰, a cycloalkyl or a nitrogen-containing saturated hetero ring, wherein said nitrogen-containing saturated hetero ring may be substituted with 1 or 2 substituent groups selected from the group consisting of a lower alkyl, -OH, -O-R⁰, -NH₂,
   -NR⁶-R⁹ and oxo (=O) ;
   R⁰: the same or different from one another and each represents a lower alkyl which may be substituted with one or more substituent groups selected from the group consisting of -OH, -O-C₁₋₄ alkyl, -NH₂, -NR⁶-C₁₋₄ alkyl and a halogen;
   R⁶: the same or different from one another and each represents a lower alkyl or H;
   R⁰⁰: the same or different from one another and each represents a lower alkylene;
   p: 0, 1 or 2;
   n: 0, 1 or 2;
   m: 0 or 1;
   R⁷ and R⁸: the same or different from each other and each represents -H, -R⁰, a halogen, -OH, -O-R⁰, -NH₂, -NR⁶-R⁰,
   -NR⁶-CO-R⁰, -O-R⁰⁰-OH, -O-R⁰⁰-O-R⁰, a cycloalkyl or an oxygen-containing saturated hetero ring, or R⁷ and R⁸ may together form a group selected from the group consisting of oxo (=O), =N-OH, =N-OR⁰ and tetrahydropyranylidene, or R⁷ and R⁸ may together form a lower alkylene which may be interrupted by 1 or 2 divalent groups selected from the class consisting of
   -O-, -S(O)ₚ-, -NR⁶- and -CONR⁶-, and may form a 3- to 8-membered ring together with the C atom to which they are linked;
   Z: -NH-;
   R³: -H or R⁰ ; and
   R⁴ and R⁵: the same or different from each other and each represents -H, -R⁰, -CO₂-R⁰, or -CO-R⁰, or R⁴ and R⁵ may together form a divalent group and may form a 5-membered hetero ring together with the -N-C-Z- group to which R⁴ and
   R⁵ are linked, wherein Z may be -O- or S-, and said 5-membered ring may be substituted with 1 or 2 substituent groups selected from a lower alkyl, -OH, -O-R⁰, -NH₂, -NR⁶-R⁰ and oxo (=O); the same shall apply hereinafter).

Among the compounds represented by the aforementioned
[1], preferred embodiments are derivatives and salts thereof described in the following [2] to [6].
[2] The derivative described in the aforementioned [1], wherein, in the aforementioned formula (I), R³ is -H or R⁰, and R⁴ and R⁵ are -H or R⁰.
[3] More preferably, the derivative described in the aforementioned [2], wherein, in the aforementioned formula (I), each of R³, R⁴ and R⁵ is -H.
[4a] More preferably, the derivative described in the aforementioned [3], wherein R⁷ and R⁸ may be the same or different from each other and each represents -H, -R⁰, -OH, -O-R⁰, -O-R⁰⁰-OH or -O-R⁰⁰-O-R⁰, or R⁷ and R⁸ together form oxo group.
[4b] Alternatively, the derivative described in the aforementioned [3], wherein R⁷ and R⁸ together form a "lower alkylene which may be interrupted by 1 or 2 divalent groups selected from the class consisting of -O-, -S(O)ₚ-, -NR⁶- and -CONR⁶", and form a 3- to 8-membered ring together with the C atom to which they are linked.
[5] More preferably, the derivatives described in the aforementioned [1] to [4b], which are compounds wherein n is 1 and R¹ is a lower alkyl substituted with a group selected from the class consisting of -OH, -O-C₁₋₄ alkyl, -NR⁶-C₁₋₄ alkyl and a halogen, or a halogen, -OH, -O-R⁰, -NH₂, -CN, -CHO or -NO₂, or compounds wherein n is 0.
[6] More preferably, the compound described in the aforementioned [3], wherein m is 0, or the derivatives described in the aforementioned [1] to [5], wherein m is 1 and R² is -R⁰ or a halogen.
[7] Particularly preferably, the derivative described in the aforementioned [1] selected from the following groups or a pharmaceutically acceptable salt thereof: N-(diaminomethylene)-9-hydroxy-9H-fluorene-2-carboxamide, 9-chloro-N-(diaminomethylene)-9H-fluorene-2-carboxamide, N-(diaminomethylene)-9-(hydroxyimino)-5-(hydroxymethyl)-9H-fluorene-2-carboxamide, 8-chloro-N-(diaminomethylene)-9-hydroxy-9H-fluorene-2-carboxamide, N-(diaminomethylene)-9-hydroxy-9-methyl-9H-fluorene-2-carboxamide, N-(diaminomethylene)-9-hydroxy-9-methyl-9H-fluorene-2-carboxamide (optically active substance A), N-(diaminomethylene)-9-hydroxy-9-methyl-9H-fluorene-2-carboxamide (optically active substance B), N-(diaminomethylene)spiro[1,3-dithiolane-2,9'-fluorene]-2'-carboxamide, N-(diaminomethylene)-4',5'-dihydro-3'H-spiro[fluorene-9,2'-furan]-2-carboxamide, N-(diaminomethylene)-4',5'-dihydro-3'H-spiro[fluorene-9,2'-furan]-2-carboxamide (optically active substance A), N-(diaminomethylene)-4',5'-dihydro-3'H-spiro[fluorene-9,2'-furan]-2-carboxamide (optically active substance B), N-(diaminomethylene)spiro[cyclopropane-1,9'-fluorene]-2'-carboxamide, N-(diaminomethylene)-9-methoxy-9-methyl-9H-fluorene-2-carboxamide, N-(diaminomethylene)-9-ethyl-9-methoxy-9H-fluorene-2-carboxamide, N-(diaminomethylene)-5-fluoro-9-hydroxy-9-methyl-9H-fluorene-2-carboxamide, N-(diaminomethylene)-5-fluoro-9-hydroxy-9-methyl-9H-fluorene-2-carboxamide (optically active substance A), N-(diaminomethylene)-5-fluoro-9-hydroxy-9-methyl-9H-fluorene-2-carboxamide (optically active substance B), N-(diaminomethylene)-5'-fluorospiro[1,3-dithiolane-2,9'-fluorene]-2'-carboxamide and N-(diaminomethylene)-5-fluoro-9-methoxy-9-methyl-9H-fluorene-2-carboxamide.

Also, the invention relates to a pharmaceutical composition comprising a fluorene derivative represented by the aforementioned general formula (I) or a pharmaceutically acceptable salt thereof and a pharmaceutically acceptable carrier. Preferred is the aforementioned pharmaceutical composition which is a 5-HT_{2B} receptor and 5-HT₇ receptor dual antagonist, and more preferred is the aforementioned pharmaceutical composition which is a prophylactic antimigraine agent.

In addition, other embodiments are use of the fluorene derivative represented by the general formula (I) or a pharmaceutically acceptable salt thereof described in [1] to [7] for producing a prophylactic antimigraine agent, and a method for preventing migraine, which comprises administering an effective amount of the fluorene derivative represented by the general formula (I) or a pharmaceutically acceptable salt thereof described in [1] to [7] to a mammal.

### Brief Description of the Drawings

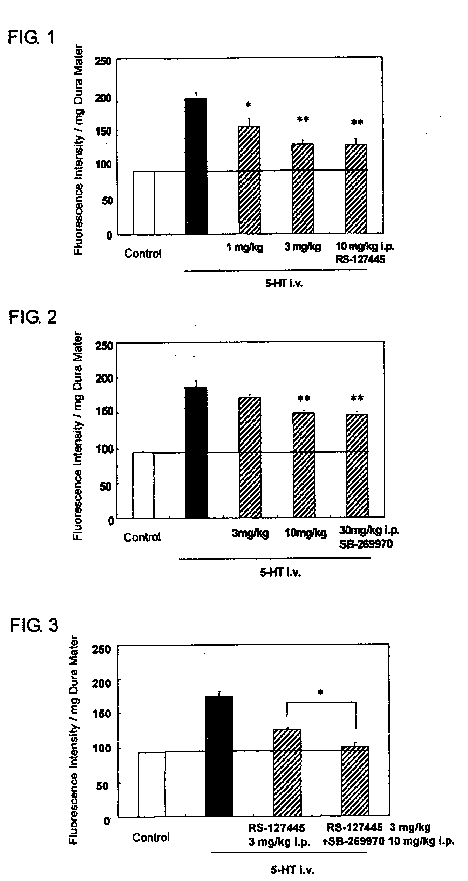

Fig. 1 is a graph showing a result of measuring leaked amount of protein at the time of RS-127445 administration, carried out by the test method (4) using a guinea pig migraine model. Statistical test was carried out by the Dunnett's test, and * means probability value is less than 5%, and ** means that is less than 1%.
Fig. 2 is a graph showing a result of measuring leaked amount of protein at the time of SB-269970 administration, carried out by the test method (4) using a guinea pig migraine model. Statistical test was carried out by the Dunnett's test, and ** means probability value is less than 1%.
Fig. 3 is a graph showing a result of measuring leaked amount of protein at the time of RS-127445 and SB-269970 simultaneous administration, carried out by the test method (4) using a guinea pig migraine model. Statistical test was carried out by the t-test, and * means probability value is less than 5%.

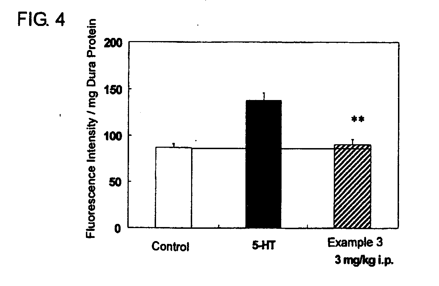

Fig. 4 is a graph showing a result of measuring leaked amount of protein at the time of the administration of the compound of Example 3, carried out by the test method (4) using a guinea pig migraine model. Statistical test was carried out by the t-test, and * means probability value is less than 5%.

### Best Mode for Carrying Out the Invention

The following describes the invention in detail.

In the definition of the general formula in this description, unless otherwise noted, the term "lower" means a straight or branched carbon chain having from 1 to 6 carbon atoms (abbreviated as "C₁₋₆"). Accordingly, the "lower alkyl" is an alkyl group with a carbon number C₁₋₆, and methyl, ethyl, propyl, isopropyl, butyl, isobutyl and tert-butyl are preferable.

The "lower alkenyl" is a C₂₋₆ alkenyl group, and vinyl, allyl, 1-propenyl, isopropenyl, 1-butenyl, 2-butenyl and 3-butenyl groups are desirable. The "lower alkynyl" is a C₂₋₆ alkynyl group, and ethynyl, 1-propynyl, 2-propynyl, 1-butynyl, 2-butynyl, 3-butynyl and 1-methyl-2-propynyl groups are preferable.

Preferred as the "lower alkylene" are methylene, ethylene, propylene, butylene and the like straight chain alkylene and methylmethylene and the like branched alkylene. Methylene, ethylene and propylene are particularly preferable.

The "halogen" means F, Cl, Br or I.

The "cycloalkyl" is a C₃₋₁₀ cycloalkyl group which may have a bridge, and preferred are cyclopropyl, cyclopentyl and cyclohexyl groups.

The "nitrogen-containing saturated hetero ring" is a 5- to 8-membered saturated or partially unsaturated monocyclic hetero ring which contains one N atom and may further contain one hetero atom selected from N, S and O, and preferred are pyrrolidinyl, piperidinyl, piperazinyl, azepanyl, diazepanyl, morpholinyl, thiomorpholinyl and tetrahydropyridyl groups.

The "oxygen-containing saturated hetero ring" is a 5-to 8-membered saturated or partially unsaturated monocyclic hetero ring which contains one O atom and may further contain one N atom, and preferred are tetrahydrofuranyl, tetrahydropyranyl, dihydropyranyl and morpholinyl groups.

The term "which may be substituted" means "no substitution" or "possession of 1 to 5 of the same or different substituent groups".

For example, the "a lower alkyl which may be substituted with halogen" is a lower alkyl substituted with one or more halogen atoms, in addition to the aforementioned lower alkyl, and preferred are C₁₋₂ alkyl having one to five of F, more preferred are fluoromethyl, difluoromethyl and trifluoromethyl.

The "lower alkylene which may be interrupted by 1 or 2 divalent groups selected from the class consisting of -O-, -S(O)ₚ-, -NR⁶- and -CONR⁶-" means a lower alkylene or a group in which 1 or 2 of a group selected from the class consisting of -O-, -S(O)ₚ-, -NR⁶- and -CONR⁶- are inserted into somewhere along or a terminus of the lower alkylene. For example, -(CH₂)₂-, -(CH₂)₃-, -(CH₂)₃-, -(-CH₂)₃-O-, -(CH₂)₂-O-(CH₂)₂-, -(CH₂)₂-S(O)-(CH₂)₂-, -(CH₂)₂-N (CH₃)- (CH₂)₂-, -O-(CH₂)₂-O-, -S-(CH₂)₂-S-, -CH₂-S-CH₂-, or CH₂CONHCH₂- or the like groups may be cited, and preferred of which is -(CH₂)₄-, -S-(CH₂)₂-S-, -(CH₂)₂-O-(CH₂)₂- or -(CH₂)₃-O-.

According to this description, the term "prophylactic antimigraine agent" means an agent or pharmaceutical composition prescribed for a patient diagnosed as a migraine patient who periodically shows symptoms of migraine, which is administered before onset of the illness in order to reduce the frequency or degree of the pain.

The "antagonist" means an agent which reduces an agonistic activity by antagonizing the agonist actions.

The "binding affinity" means an ability to bind to a part of a receptor, and evaluation of this is carried out by comparing Ki values calculated by an in vitro receptor binding test, or, as occasion demands, IC₅₀ values calculated by the receptor binding test carried out under the same conditions, as is described later(test protocol). In this connection, when the IC₅₀ value cannot be calculated in the receptor binding test due to insufficient inhibitory activity at a certain concentration, IC₅₀ value of the compound is regarded sometimes as said concentration or more.

When binding affinity of the compound of the invention for 5-HT_{2B} receptor and 5-HT₇ receptor is "selective" in comparison with other receptors, it means that the binding affinity for said receptors is relatively high in comparison with the binding affinity for "other receptors". According to the invention, the term "selective" means a case in which the Ki value or IC₅₀ value representing binding affinity for said receptors is 1/10 or less in comparison with the value for "other receptors", and this value is more preferably 1/50 or less, further preferably 1/100 or less, more further preferably 1/500 or less, and particularly preferably 1/1000 or less.

In this case, the "other receptors" are other receptors described in the reports on the existing non-selective serotonin receptor antagonists, which are receptors participate in the particularly undesirable actions. Accordingly, preferable as the compounds of the invention are compounds selective against α₁, M₁ and D₂ receptors, and are more desirably compounds selective against α₁, M₁, D₂, 5-HT_{1A}, 5-HT_{1B}, 5-HT_{2A}, 5-HT_{2C}, 5-HT₃, 5-HT₄ and 5-HT₆ receptors.

As the pharmaceutically acceptable salt of the compound (I) of the invention, illustratively, acid addition salts with hydrochloric acid, hydrobromic acid, hydroiodic acid, sulfuric acid, nitric acid, phosphoric acid and the like inorganic acids, formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid and the like organic acid, and the like may be exemplified. In addition, there is a case in which a salt with a bases is formed depending on the kind of substituent groups, and its examples include salts with inorganic bases containing sodium, potassium, magnesium, calcium, aluminum and the like metals, or with methylamine, ethyl amine, ethanolamine, lysine, ornithine and the like organic bases, ammonium salts and the like.

There is a case in which geometrical isomers and tautomers exist in the compound (I) of the invention. For example, the following tautomers exist in a compound in which R³ in the formula (I) is -H.

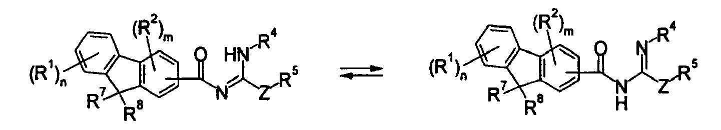

The invention includes one of such tautomers or their mixture.

Also, isomers based on asymmetric carbon atom exist in the compound of the invention in some cases. The invention includes mixtures and isolated forms of these optical isomers. According to this description, those in which (optically active substance A) or (optically active substance B) is added to the rear of the name of a compound represent one of respective optical isomers. In this case, a compound as an optically active substance A means an isomer having a relatively short retention time (quickly eluted), and a compound as an optically active substance B means an isomer having a relatively long retention time (slowly eluted), under the high performance liquid chromatography analysis conditions described in the tables of Examples (cf. Examples 56a, 56b, 59a, 59b, 60a and 60b).

Also, the compound (I) of the invention forms N-oxide in some cases depending on the kind of substituent groups, and these N-oxide compounds are also included. In addition, various hydrates and solvates and polymorphic substances are also included.

In this connection, all of the compounds which are metabolized in the living body and converted into the compound (I) or a salt thereof, so-called prodrugs, are also included in the compound (I) of the invention. As the groups for forming these prodrugs, the groups described in Prog. Med. 5:2156 - 2161 (1985) and the groups described in "Iyakuhin no Kaihatsu (Development of Medicines)" vol. 7 Bunshi Sekkey (Molecular Design) 163 - 198, published in 1990 by Hirokawa Shoten may be exemplified.

### (Production methods)

The compounds of the invention and pharmaceutically acceptable salts thereof may be produced by employing various conventionally known synthesis methods, making use of characteristics based on their core structure or the kind of substituent groups. In this connection, depending on the kind of functional group, it is technically effective in some cases to protect said functional group with an appropriate protecting group, or replace said functional group with a group which can be easily converted into said functional group, at a stage of starting compounds or intermediates. Examples of such a functional group include amino group, hydroxyl group, carbonyl group, carboxyl group and the like, and the protecting groups described in "Protective Groups in Organic Synthesis (3rd Edition, 1999, John Wiley & Sons)" edited by T.W. Greene and P.G. Wuts may for example be cited as their protecting groups which may be optionally used in response to the reaction conditions. According to such a method, the compound of interest may be obtained by introducing said protecting group and carrying out the reaction, and then removing the corresponding protecting group or converting it into a desired group.

In addition, similar to the case of the aforementioned protecting group, prodrugs of the compound of the invention may be produced by introducing a specific group at a stage of starting compounds or intermediates, or carrying out a reaction using the obtained compound of the invention. The reaction may be carried out by employing general esterification, amidation, dehydration and the like conventional methods known to those skilled in the art.

Typical production methods of the compound of the invention are described in the following.

### (Production Method 1)

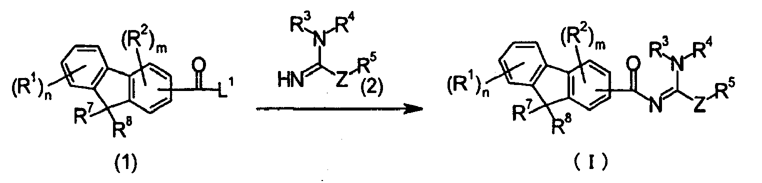

(In the formula, L¹ represents -OH or -O-lower alkyl, or halogen, -O-methanesulfonyl, -O-p-toluenesulfonyl or the like leaving group.)

The compound (I) of the invention may be produced by subjecting a compound represented by (1) which is a carboxylic acid or a reactive derivative thereof and an amine derivative (2) to an amidation reaction.

When a free carboxylic acid in which L¹ in the starting compound (1) is OH is used, a method in which the compound (1) and amine derivative (2) are dehydration condensed in the presence of a condensing agent is used. In that case, it is preferable to use N,N'-dicyclohexylcarbodiimide (DCC), 1-[3-(dimethylamino)propyl]-3-ethylcarbodiimide (WSC), 1,1'-carbonyldiimidazole (CDI), 2-(1H-benzotriazol-1-yl)-1,1,3,3-tetramethyluronium hexafluorophosphate (HBTU), diphenylphosphoryl azide (DPPA), phosphorus oxychloride or the like condensing agent and further, as occasion demands, an additive agent (e.g., N-hydroxysuccinimide (HONSu), 1-hydroxybenzotriazole (HOBt) or the like).

The reaction is carried out by using the compound (1) and amine derivative (2) in equivalent amounts or one of them in an excess amount, and using the condensing agent in equivalent amount or excess amount based on the carboxylic acid. The reaction may be carried out under cooling to heating, preferably at -20°C to 60°C, in a inert solvent such as benzene, toluene, xylene or the like aromatic hydrocarbon, dichloromethane, 1,2-dichloroethane, chloroform or the like halogenated hydrocarbon, diethyl ether, tetrahydrofuran (THF), dioxane, dimethoxyethane (DME) or the like ether, N,N-dimethylformamide (DMF), dimethyl sulfoxide (DMSO), ethyl acetate, acetonitrile, water or the like, or in a mixed solvent thereof.

When a compound in which L¹ in the starting compound (1) is a leaving group, namely a reactive derivative of the carboxylic acid, is used, the reaction is carried out using the compound (1) and amine derivative (2) in equivalent amounts or one of them in an excess amount, under the same conditions of the aforementioned case of the use of free carboxylic acid. As the reactive derivative of carboxylic acid to be used in this case, an acid halide (acid chloride, acid bromide or the like), an acid anhydride (phenyl chloroformate, a mixed anhydride prepared from p-toluenesulfonic acid or isovaleric acid or the like, or symmetric acid anhydride of itself), an activated ester (an ester which may be prepared using phenol which may be substituted with nitro group, fluorine atom or the like electron withdrawing group, HOBt, HONSu or the like), a lower alkyl ester and the like may be exemplified, and each of them may be produced from the carboxylic acid using corresponding reaction obvious to those skilled in the art. Depending on the kind of reactive derivatives, it is advantageous in some cases, for smoothly progressing the reaction, to carry out the reaction in the presence of a base (triethylamine, diisopropylethylamine, N-methylmorpholine, pyridine, 4-(N,N-dimethylamino)pyridine or the like organic base, or sodium bicarbonate or the like inorganic base or the like). Pyridine may also serve as the solvent. In this connection, when a lower alkyl ester is used as the reactive derivative, it is preferable to carry out the reaction from under room temperature to under heat reflux.

### (Production Method 2)

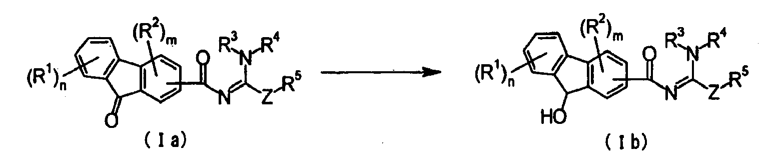

Among compounds (I) of the invention, a compound (Ib) wherein -CR⁷R⁸- is represented by -CH(OH)- may be produced by subjecting a compound (Ia) of the invention in which said part is carbonyl group to a reduction reaction.

The reaction is carried out by treating the compound (Ia) with equivalent amount or an excess amount of a reducing agent. As the reducing agent, sodium borohydride, diisobutyl aluminum hydride or the like hydride reducing agent or a reducing agent described in "Comprehensive Organic Transformations" edited by Richard C. Larock (1989, VCH Publishers, Inc.) is used. The reaction is carried out using an aromatic hydrocarbon, an ether, DMF, DMSO, an alcohol (methanol, ethanol or the like) or water, or a mixture thereof, as the solvent, and performed under cooling to under heating, preferably at -20°C to room temperature.

When the compound (I) of the invention has various side chains and substituent groups, these compounds may be easily synthesized using the compound of the invention or a production intermediate thereof as the material, by using a method obvious to those skilled in the art or a modified method thereof. A such an example, a reaction in which substituent groups of the compounds obtained by the production methods 1 and 2 are further converted may be cited, and the reactions shown below may be employed.

A compound in which R⁷ and R⁸ in the formula (I) together represent =N-OR⁰ may be produced by carrying out dehydration condensation of the compound (Ia) of the invention wherein said moiety is oxo group and NH₂-OR⁰.

A compound in which one of R⁷ and R⁸ in the formula (I) is a halogen may be produced by subjecting the compound (Ib) of the invention wherein said moiety is -CH(OH)- to a halogenation reaction.

A compound in which R¹ in the formula (I) is -NH₂ may be produced by subjecting the compound of the invention wherein said moiety is -NO₂ to a reduction.

### (Production of starting compounds)

The starting compound (1) in the aforementioned production methods may be produced making use of conventionally known methods. 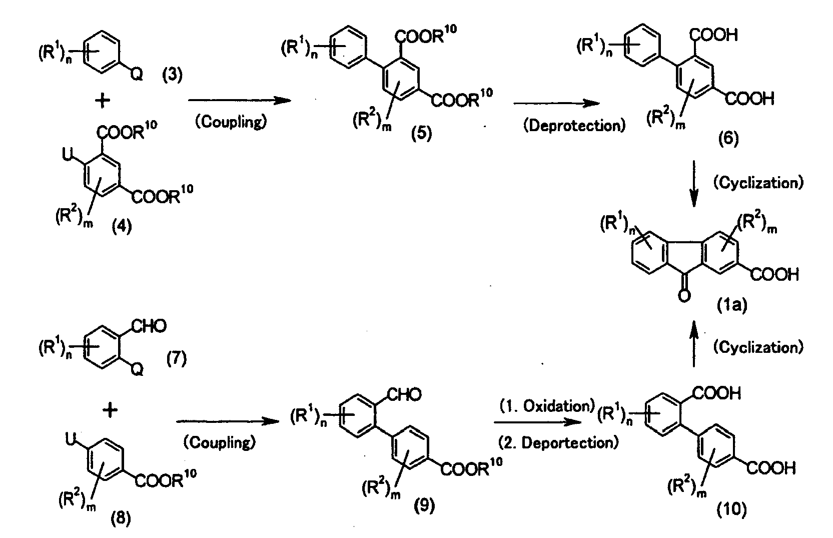 (In the reaction scheme, either one of Q and U is -Br, -Cl, -I or O-SO₂-CF₃, and the other is -B(OH)₂ or a B(O-lower alkyl)₂. R¹⁰ represents a lower alkyl or benzyl or the like protecting group.)

The compound (1a) in which R⁷ and R⁸ in the formula (1) together form oxo group and L¹ is hydroxyl group may be produced by the aforementioned reaction pathway.

In this reaction pathway, particularly the coupling reaction may be carried out by the method described in Synth. Commun., 11, 513 - 519 (1981), Synlett, 6, 829 - 831 (2000) or Chem. Lett., 1405 - 1408 (1989). The conventional intramolecular Friedel-Crafts' reaction may be used in the cyclization reaction, and the method described in J. Am. Chem. Soc., 63, 1948 (1941) may be exemplified. The oxidation reaction may be carried out at room temperature or under heating in DMF, methanol, water or the like solvent or in a mixture of them, using silver oxide, pyridinium dichromate, sodium chlorite or the like oxidizing agent. 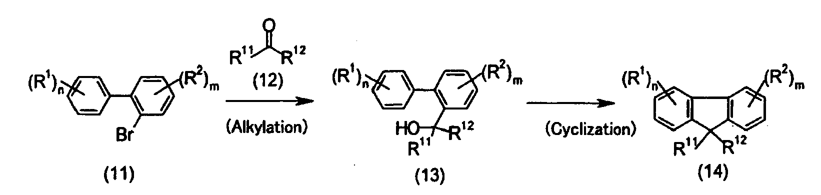 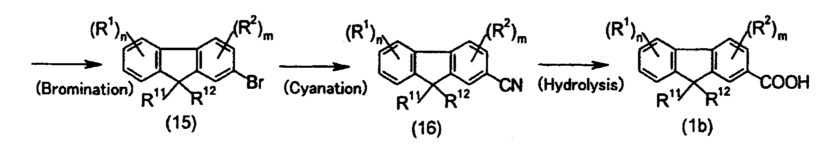 (In the reaction scheme, R¹¹ and R¹² represent a lower alkyl which may be substituted, or R¹¹ and R¹² together form a lower alkylene which may be interrupted by -O- or -NR⁶-.)

The compound (1b) in which at least one of R⁷ and R⁸ in the formula (1) is an alkyl group may be produced by bromination of the aromatic ring of the fluorene (14) which is obtained with reference to the method described in J. Am. Chem. Soc., 63, 1948 (1941), making this into cyano group and then converting it into carboxyl group. In this case, the bromination may be carried out in accordance with the method described in "Orgnikum" edited by H. Becher, p. 189, 1973, and the cyanation with that in J. Org. Chem., 26, 2522 (1961). 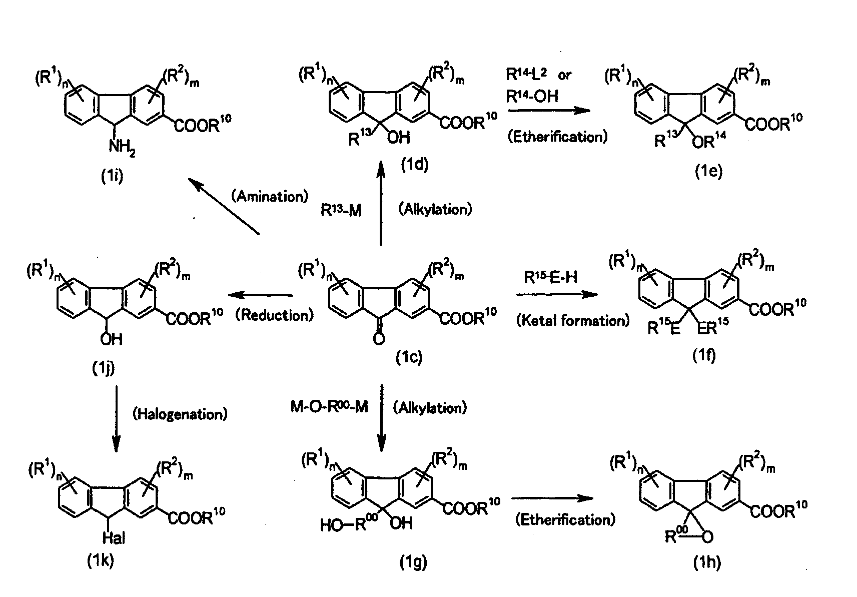 (In the reaction scheme, R¹³ and R¹⁴ represent a lower alkyl, R¹⁵ represents a lower alkyl or two R¹⁵ groups together represent a lower alkylene, M represents lithium ion, magnesium ion or the like counter cation of an organometalic reagent, E represents -O- or S-, L² represents halogen, -O-methanesulfonyl, O-p-toluenesulfonyl or the like leaving group, and Hal represents a halogen.)

Among the starting compounds (1), a compound in which at least one of R⁷ and R⁸ has various substituent groups may be easily produced from a 9-keto compound (1c) using each of the alkylation, etherification, ketal formation, amination, reduction and halogenation, or by a combination thereof.

Particularly, the alkylation may be carried out using Grignard reagent, organic lithium reagent, organic cerium reagent or the like organic metal reagent. The etherification for producing compound (1e) from (1d) is carried out using R¹⁴-L² as the alkylation agent, in the presence of sodium hydride, potassium hydride, potassium tert-butoxide, silver oxide or the like base. Alternatively, there is a case in which this alkylation is carried out under an acidic condition using R¹⁴-OH, and the reaction is carried out at room temperature to heating in methanol, ethanol, benzene, toluene, xylene or the like solvent, using toluenesulfonic acid or the like acid catalyst, iron nitrate or iron perchlorate or the like Lewis acid.

In the aforementioned reaction pathway, each of the alkylation, amination and reduction may also be carried out using the carboxyl compound (1a) instead of the starting compound (1c). In addition, regarding each of the compounds (1c) to (1k), it may be converted into corresponding carboxyl compound through deprotection of the COOR¹⁰ group.

The compound (I) produced in this manner is isolated and purified directly as its free form or as a salt by subjecting it to a salt formation treatment in the usual way. The isolation and purification are carried out by employing general chemical operations such as extraction, concentration, evaporation, crystallization, filtration, recrystallization, various chromatographic treatments and the like.

Various isomers may be isolated in the usual way making use of a difference in physicochemical property between isomers. For example, optical isomers may be separated and purified by a method in which a racemic compound is made into a diastereomeric mixture of its salts with an optically active organic acid (tartaric acid or the like) and then subjected to fractional recrystallization, or a column chromatography packed with chiral stationary phase or the like technique. In addition, an optically active compound may also be produced using an appropriate optically active compound as the material. In this connection, a mixture of diastereomers may also be separated by fractional crystallization, chromatography and the like.

### Industrial Applicability

Since the compound of the invention has excellent migraine preventive effect and simultaneously and selectively inhibits both 5-HT_{2B} and 5-HT, receptors, this is useful as a prophylactic antimigraine agent having excellent effect and high safety with reduced side effects caused by receptor antagonism of other than 5-HT_{2B} and 5-HT₇ receptors as reported in the existing agents.

In addition, therapeutic or preventive effect of the compound of the invention are expected also on pulmonary hypertension, anxiety and sleeplessness upon which of 5-HT_{2B} selective inhibitors and 5-HT, selective inhibitors show the efficacy.

Usefulness of the compound (I) of the invention was confirmed by the following tests.

### (1) Binding test of 5-HT_{2B} receptor

### (i) Preparation of membrane preparation

A human 5-HT_{2B} receptor expressing cell was prepared in accordance with a reference (FEBS Letters (1994) 342, 85 - 90). HEK293-ENBA cell was used as the gene transferring cell.

Cultured HEK293-EBNA cells expressing human 5-HT_{2B} receptor were washed with PBS(-). The cells were scraped in the presence of PBS(-), and the cells were recovered by centrifugation (1,000 rpm, 10 min, 4°C). They were homogenized using Polytron (PTA 10-TS) in the presence of 5 mM Tris-HCl (pH 7.4) buffer and centrifuged (40,000 x g, 10 min, 4°C). They were suspended using a homogenizer in the presence of 50 mM Tris-HCl (pH 7.4) buffer. They were subjected to centrifugation (40,000 x g, 10 min, 4°C), suspended in 50 mM Tris-HCl (pH 7.4) and stored at -80°C.

### (ii) Receptor binding test

A total volume of 500 µl containing 50 mM Tris-HCl-4 mM CaCl₂ (pH 7.4) buffer, the human 5-HT_{2B} receptor expressing HEK293-EBNA cell membrane preparation and a radio ligand [³H]Mesulergine (3.1 TBq/mmol) was incubated at 25°C for 1 hour. The compound was dissolved in 100% DMSO and diluted to respective concentrations. Nonspecific binding was defined as the binding quantity in the presence of 1 µM ritanserin, and the result of subtracting the nonspecific binding quantity from the total binding quantity was defined as the specific binding quantity. This was mixed with 4 ml of 50 mM Tris-HCl buffer (pH 7.4) and filtered under a reduced pressure using a GF/B glass filter, and the filter was washed (4 ml x 3) with the same buffer. The glass filter was soaked in 5 ml of a liquid scintillator (Aquasol-2) and the radioactivity was measured using a liquid scintillation counter. Concentration of the compound which inhibits 50% of the receptor binding, IC₅₀ value, was obtained by nonlinear regression analysis using SAS (ver. 6.11), and the Ki value which represents its affinity for the receptor was calculated using the formula of Cheng & Prussoff; Ki = IC₅₀/(1 + [L]/[Kd]) ([L]: ligand concentration, [Kd]: dissociation constant).

The compound of Example 3 which is described later showed a Ki value of 1.8 nM. Also, the compounds of Examples 4, 7, 8, 34, 38, 56, 56a, 56b, 59, 60, 60a, 60b, 63, 71, 72, 77, 78a, 78b, 85 and 87 showed Ki values of from 0.1 to 350 nM.

### (2) Binding test of 5-HT, receptor

### (i) Preparation of membrane preparation

A human 5-HT, receptor expressing cell was prepared in accordance with references (J. Biol. Chem. (1993) 268, 31, 23422 - 23426, Br. J. Phaemacol. (1997) 122, 126 - 132). CHO cell was used as the gene transferring cell.

Cultured CHO cells expressing human 5-HT₇ receptor were washed with PBS(-). The cells were scraped in the presence of PBS(-), and the cells were recovered by centrifugation (1,000 rpm, 10 min, 4°C). They were homogenized using Polytron (PTA 10-TS) in the presence of 5 mM Tris-HCl (pH 7.4) buffer and centrifuged (40,000 x g, 10 min, 4°C). They were suspended using a homogenizer in the presence of 50 mM Tris-HCl (pH 7.4) buffer. They were subjected to centrifugation (40,000 x g, 10 min, 4°C), suspended in 50 mM Tris-HCl (pH 7.4) and stored at -80°C.

### (ii) Receptor binding test

A total volume of 500 µl containing 50 mM Tris-HCl-4 mM CaCl₂ (pH 7.4) buffer, the human 5-HT₇ receptor expressing CHO cell membrane preparation and a radio ligand [³H]5-HT (3.40 TBq/mmol) was incubated at 25°C for 1 hour. The compound was dissolved in 100% DMSO and diluted to respective concentrations. Nonspecific binding was defined as the binding quantity in the presence of 10 µM metergoline, and the result of subtracting the nonspecific binding quantity from the total binding quantity was defined as the specific binding quantity. This was mixed with 4 ml of 50 mM Tris-HCl buffer (pH 7.4) and filtered under a reduced pressure using a GF/B glass filter, and the filter was washed (4 ml x 3) with the same buffer. The glass filter was soaked in 5 ml of a liquid scintillator (Aquasol-2) and the radioactivity was measured using a liquid scintillation counter. Concentration of the compound which inhibits 50% of the receptor binding, IC₅₀ value, was obtained by nonlinear regression analysis using SAS (ver. 6.11), and the Ki value which represents its affinity for the receptor was calculated using the formula of Cheng & Prussoff; Ki = IC₅₀/(1 + [L]/[Kd]) ([L]: ligand concentration, [Kd]: dissociation constant).

The compound of Example 3 which is described later showed a Ki value of 17.6 nM. Also, the compounds of Examples 4, 7, 8, 34, 38, 56, 56a, 56b, 59, 60, 60a, 60b, 63, 71, 72, 77, 78a, 78b, 85 and 87 showed Ki values of from 0.4 to 310 nM.

### (3) Affinity for other receptors

Affinity of the compound of Example 3 for 5-HT_{1A}, 5-HT_{1B}, 5-ET_{2A}, 5-HT_{2C}, 5-HT₃, 5-ET₄, 5-HT₆, α₁, M₁ and D₂ receptors was verified using conventionally known techniques (Journal of Neurochemistry (1986) 47, 529 - 540; Molecular Pharmacology (1982) 21, 301 - 314; European Journal of Pharmacology (1985) 106, 539 - 546; Journal of Pharmacology Experimental Therapy (1992) 263, 1127 - 1132; British Journal of Pharmacology (1993) 109, 618 - 624; Molecular Pharmacology (1993) 43, 320 - 327; Molecular Pharmacology (1989) 35, 324 - 330; Cellular Molecular Neurobiology (1988) 8, 181 - 191; European Journal of Pharmacology (1988) 173, 177 - 182). As a result, IC₅₀ value of this compound was 1 µM or more on all of the 5-HT_{1A}, 5-HT_{1B}, 5-HT_{2A}, 5-HT_{2C}, 5-HT₃, 5-HT₄, 5-HT₆, α₁, M₁ and D₂ receptors. In addition, when the affinity for each of α₁, M₁ and D₂ receptors was verified using the aforementioned technique on the compounds of Examples 56, 59, 60, 71, 72, 77 and 85 which are described later, 5-HT_{2B} and 5-HT₇ receptor selectivity of these compounds against α₁, M₁ and D₂ receptors was 100 times or more.

Based on the above results, it was shown that the Example compounds of the present invention have selective binding affinity for both of the 5-HT_{2B} and 5-HT₇ receptors.

In this connection, affinities of each of RS-127445 (2-amino-4-(4-fluoronaphth-1-yl)-6-isopropylpyrimidine; see WO 97/44326 for its production method) and SB-269970 ((R)-3-(2-(2-(4-methylpiperidin-1-yl)ethyl)pyrrolidine-1-sulfonyl)phenol; see WO 97/48681 for its production method) described in the following test method (4) for respective receptors are conventionally known, and regarding the RS-127445, it has been reported that said compound has a pKi value of 9.5 for 5-HT_{2B} receptor, and is 1000 times more 5-HT_{2B} receptor selective against 5-HT_{1A}, 5-HT_{1B}, 5-HT_{2A}, 5-HT_{2C}, 5-HT₃, 5-HT₆, 5-HT₇, α₁, M₁ and D₂ receptors. Also, regarding the SB-269970, it has been reported, for example in J. Med. Chen. (2000) 43, 342 - 345, that said compound has a pKi value of 8.9 for 5-HT_{2B} receptor, and is 250 times more 5-HT₇ receptor selective against 5-HT_{1A}, 5-HT_{1B}, 5-HT_{2A}, 5-HT_{2B}, 5-HT_{2C}, 5-HT₄, 5-HT₆, α₁ and D₂ receptors.

### (4) Preventive effect in guinea pig migraine model

It has been suggested that an inflammatory protein leaked from the dural blood vessel caused by 5-HT is concerned in the onset of migraine. In this test system, migraine preventive effect was evaluated by measuring this leaked protein in the presence of a compound to be tested, and this was carried out by partially modifying the method described in Rachel A. Spokes and Vicki C. Middlefell, European Journal of Pharmacology (1995) 281, 75 - 79.

Hartley male guinea pig (250 - 350 g) was anesthetized by peritoneal administration (i.p.) of urethane (1.5 g/kg). By applying simple canulation to a saphena, 50 mg/kg of a fluorescent protein (FITC-BSA) was intravenously administered (i.v.) and 5 minutes thereafter, physiological saline or 1 µM of 5-HT was intravenously administered. By carrying out perfusion with physiological saline 15 minutes thereafter, blood was washed out. Each of RS-127445, SB-269970 and the compound of Example 3 was intraperitoneally administered, and other Example compounds orally, 30 minutes before administration of the fluorescent protein. By detaching the skull, dura mater was took out and incubated at 37°C for 16 hours in an Eppendorf tube in the presence of physiological saline of pH 11. Centrifugation was carried out, and the supernatant was dispensed into a plate. Fluorescence intensity was measured using a fluorescence plate reader (excitation wavelength 485 nm, absorption wavelength 530 nm). By measuring dura mater weight, fluorescence intensity per mg dural protein was calculated.

Values of the fluorescence intensity measured at the time of the administration of each compound and at the time of no administration are shown in Fig. 1 to Fig. 4. In each of them, the axis of abscissa shows dose of the compound, and the axis of ordinate fluorescence intensity per 1 mg dural blood vessel. The control indicates fluorescence intensity at the time of no addition of 5-HT, namely the reference value.

As shown in Fig. 1, the 5-HT_{2B} selective antagonistic compound RS-127445 showed an effect to reduce amount of the leaked protein at 3 mg/kg, but did not reduced to the reference value when the dose was increased from 3 mg/kg to 10 mg/kg.

In addition, as shown in Fig. 2, the 5-HT₇ selective antagonistic compound SB-269970 also showed the effect from 10 mg/kg, but did not reduced the amount of leaked protein to the reference value when the dose was increased from 3 mg/kg to 30 mg/kg.

On the other hand, as shown in Fig. 3, it was found that a synergistic effect is obtained when both compounds of RS-127445 and SB-269970 are simultaneously administered. That is, as shown in Fig. 1 and Fig. 2, it was shown that the minimum amount of both compounds showing maximum drug effect in this model is 3 mg/kg for RS-127445 and 10 mg/kg for SB-269970, but it was revealed that the amount of leaked protein is suppressed almost completely to the reference value when both compounds are simultaneously administered at the same dose. This result shows that when both functions of 5-HT_{2B} receptor and 5-HT, receptor are simultaneously inhibited, an excellent effect which cannot be attained by the selective inhibition of one of the receptors may be obtained.

This effect was the same when the compound of the invention simultaneously having selective 5-HT_{2B} receptor antagonism and 5-HT, receptor antagonism was used. That is, the compound of Example 3 which is described later almost completely suppressed the amount of leaked protein by 3 mg/kg of intraperitoneal administration as shown in Fig. 4.

In addition, the compounds of Examples 56, 56a, 59, 60, 60a, 60b, 71, 72, 77, 78b, 85 and 87 which are described later also almost completely suppressed leakage of the protein by 10 mg/kg or 30 mg/kg of oral administration.

Based on the above results, it was shown that the compound of the invention can completely inhibit leakage of the inflammatory protein by simultaneously having 5-HT_{2B} receptor antagonism and 5-HT₇ receptor antagonism. Accordingly, it is shown that the compound of the invention has a possibility of effectively inhibiting inset of migraine and has excellent migraine preventive effect in comparison with one of the selective receptor antagonists.

The pharmaceutical composition of the invention which contains one or more of the compounds represented by the general formula (I) as the active ingredient may be prepared by generally used methods using carriers, fillers and the like for medicament use conventionally used in this field. Administration may be in either embodiment of oral administration by tablets, pills, capsules, granules, powders, solutions and the like or parenteral administration by intravenous injections, intramuscular injections and the like injections, ointments, hard cream preparations, creams, jellies, cataplasmas, sprays, lotions, eye drops, eye ointments and the like external preparations, suppositories, inhalations and the like.

The solid composition for use in the oral administration according to the present invention is used in the form of tablets, powders, granules and the like. In such a solid composition, one or more active substances are mixed with at least one inert filler such as lactose, mannitol, glucose, hydroxypropylcellulose, microcrystalline cellulose, starch, polyvinyl pyrrolidone, aluminum magnesium silicate and the like. In the usual way, the composition may contain inert additives such as magnesium stearate or the like lubricant, sodium carboxymethylstarch or the like disintegrating agent, and a solubilizing agent. As occasion demands, tablets or pills may be coated with a sugar coating or a film of a gastric or enteric coating agent.

The liquid composition for oral administration includes pharmaceutically acceptable emulsions, solutions, suspensions, syrups, elixirs and the like, and a generally used inert solvent such as purified water or ethanol may be used. In addition to the inert solvent, this composition may also contain a solubilizing agent, a moistening agent, a suspending agent and the like auxiliary agents, as well as sweeteners, flavors, aromatics and antiseptics.

The injections for parenteral administration includes aseptic aqueous or non-aqueous solutions, suspensions and emulsions. Examples of the aqueous solvent include distilled water for injection and physiological saline. Examples of the non-aqueous solvent include propylene glycol, polyethylene glycol, olive oil or the like plant oil, ethanol or the like alcohol, polysorbate 80 (official name listed in the Pharmacopoeia of Japan) and the like. Such a composition may further contain a tonicity agent, an antiseptic, a moistening agent, an emulsifying agent, a dispersing agent, a stabilizing agent and a solubilizing agent. These compositions are sterilized by filtration through a bacteria retaining filter, blending of a germicide or irradiation. Alternatively, they may be used by firstly making into sterile solid compositions and dissolving or suspending them in sterile water or a sterile solvent for injection prior to their use.

When the compound of the invention is administered as a prophylactic antimigraine agent, it is generally administered preventively before onset of migraine. Accordingly, it is preferable to take it continuously while the onset occur frequently.

Daily dose of the compound of the invention is generally from about 0.001 to 50 mg/kg, preferably from 0.01 to 30 mg/kg, more preferably from 0.05 to 10 mg/kg, per body weight in the case of oral administration, or when intravenously administered, the daily dose is from about 0.0001 to 10 mg/kg, preferably from 0.001 to 1.0 mg/kg, per body weight, and this is administered by dividing into 1 to several doses per day. The dose is optionally decided by taking into consideration symptoms, age, sex and the like of each case.

### Examples

The following describes compounds of the invention further in detail with reference to Examples. In this connection, since novel compounds are also included in the starting compounds for the compounds of the invention, their production methods are described as Reference Examples.

### Reference Example 1-a

Diethyl 2'-methylbiphenyl-2,4-dicarboxylate was obtained by allowing 4-bromoisophthalic acid diethyl ester to react with 2-methylphenylboronic acid, sodium carbonate and tetrakistriphenylphosphine palladium under heating in toluene-ethanol-water. FAB-MS: 313 (M + H)⁺.

### Reference Example 1-b

2'-Methylbiphenyl-2,4-dicarboxylic acid was obtained by treating ethanol solution of diethyl 2'-methylbiphenyl-2,4-dicarboxylate with 1 M sodium hydroxide. FAB-MS: 257 (M + H)⁺.

### Reference Example 1-c

5-Methyl-9-oxo-9H-fluorene-2-carboxylic acid was obtained by heating 2'-methylbiphenyl-2,4-dicarboxylic acid in polyphosphoric acid. FAB-MS: 239 (M + H)⁺.

### Reference Example 2

3'-Methylbiphenyl-2,4-dicarboxylic acid was treated in the same manner as in Reference Example 1-c, and then the thus obtained solid was heated in ethanol in the presence of concentrated sulfuric acid to carry out esterification. After treatment of the reaction, this was separated and purified by a silica gel column chromatography to obtain ethyl 6-methyl-9-oxo-9H-fluorene-2-carboxylate [FAB-MS: 267 (M + H)⁺] and ethyl 8-methyl-9-oxo-9H-fluorene-2-carboxylate [FAB-MS: 267 (M + H)⁺], respectively.

### Reference Example 3

In the same manner as in Reference Example 1-a, ethyl 3-chloro-2'-formylbiphenyl-4-carboxylate was produced from ethyl 4-bromo-2-chlorobenzoate and 2-formylphenylboronic acid. FAB-MS: 288 (M)⁺.

3'-Chloro-4'-(ethoxycarbonyl)biphenyl-2-carboxylic acid was obtained by allowing ethyl 3-chloro-2'-formylbiphenyl-4-carboxylate to react with sodium perchlorate, sodium dihydrogenphosphate and 2-methyl-2-butene in tert-butanol-acetonitrile-water at room temperature. FAB-MS: 305 (M + H)⁺.

Thereafter, in the same manner as in Reference Example 1-c and Reference Example 2, ethyl 1-chloro-9-oxo-9H-fluorene-2-carboxylate [FAB-MS: 287 (M + H)⁺] and ethyl 3-chloro-9-oxo-9H-fluorene-2-carboxylate [FAB-MS: 287 (M + H)⁺] were produced, respectively.

### Reference Example 4-a

9-Hydroxy-9-methyl-9H-fluorene-2-carboxylic acid was obtained by allowing 9-oxo-9H-fluorene-2-carboxylic acid to react with methyl lithium in THF at from -20°C to 0°C. FAB-MS: 239 (M - H)⁻.

### Reference Example 4-b

Methyl 9-hydroxy-9-methyl-9H-fluorene-2-carboxylate was obtained by allowing 9-hydroxy-9-methyl-9H-fluorene-2-carboxylic acid to react with sodium bicarbonate and methyl iodide in DMF at room temperature. FAB-MS: 255 (M + H)⁺.

### Reference Example 4-c

Methyl 9-methoxy-9-methyl-9H-fluorene-2-carboxylate was obtained by Allowing methyl 9-hydroxy-9-methyl-9H-fluorene-2-carboxylate to react with iron nitrate in methanol under heating. FAB-MS: 269 (M + H)⁺.

### Reference Example 5

Methyl 9-methoxymethyl-9-methyl-9H-fluorene-2-carboxylate was obtained by allowing methyl 9-hydroxy-9-methyl-9H-fluorene-2-carboxylate to react with sodium hydride and methoxymethyl chloride in DMF at room temperature. FAB-MS: 298 (M)⁺.

### Reference Example 6-a

In THF, methylmagnesium bromide was allowed to act upon 3-chloropropan-1-ol to form magnesium oxide, and then magnesium metal was allowed to react therewith, thereby preparing a Grignard reagent (ClMg(CH₂)₃OMgBr). This was allowed to react with 9-oxo-9H-fluorene-2-carboxylic acid in the same manner as in Reference Example 4-a, and then the thus obtained 9-hydroxy-9-hydroxypropyl-9H-fluorene-2-carboxylic acid was allowed to react with methyl iodide in the same manner as in Reference Example 4-b, thereby obtaining methyl 9-hydroxy-9-hydroxypropyl-9H-fluorene-2-carboxylate. FAB-MS: 297 (M - H)⁻.

### Reference Example 6-b

Methyl 4',5'-dihydro-3'H-spiro[fluorene-9,2'-furan]-2-carboxylate was obtained by allowing methyl 9-hydroxy-9-hydroxypropyl-9H-fluorene-2-carboxylate to undergo the reaction under heating in toluene in the presence of p-toluenesulfonic acid. FAB-MS: 281 (M + H)⁺.

### Reference Example 7-a

Methyl 8-bromomethyl-9-oxo-9H-fluorene-2-carboxylate was obtained by allowing methyl 8-methyl-9-oxo-9H-fluorene-2-carboxylate to react with N-bromosuccinimide and 2,2'-azobisisobutyronitrile under heating in carbon tetrachloride. EI-MS: 330 (M)⁺, 332 (M + 2)⁺.

### Reference Example 7-b

Methyl 8-dimethylaminomethyl-9-oxo-9H-fluorene-2-carboxylate was obtained by allowing methyl 8-bromomethyl-9-oxo-9H-fluorene-2-carboxylate to react with dimethylamine (2 M, methanol solution) and potassium carbonate at room temperature in THF. FAB-MS: 296 (M + H)⁺.

### Reference Example 8-a

Methyl 8-acetoxymethyl-9-oxo-9H-fluorene-2-carboxylate was obtained by allowing methyl 8-bromomethyl-9-oxo-9H-fluorene-2-carboxylate to react with potassium acetate at room temperature in DMF. FAB-MS: 311 (M + H)⁺.

### Reference Example 8-b

Methyl 8-hydroxymethyl-9-oxo-9H-fluorene-2-carboxylate was obtained by allowing methyl 8-acetoxymethyl-9-oxo-9H-fluorene-2-carboxylate to react with potassium carbonate at room temperature in methanol-THF. FAB-MS: 269 (M + H)⁺.

### Reference Example 8-c

Methyl 8-methoxymethyl-9-oxo-9H-fluorene-2-carboxylate was obtained by allowing methyl 8-hydroxymethyl-9-oxo-9H-fluorene-2-carboxylate to react with methyl iodide and silver oxide under heating in acetonitrile. FAB-MS: 283 (M + H)⁺.

### Reference Example 9

Methyl 9-fluoro-9H-fluorene-2-carboxylate was obtained by allowing methyl 9-oxo-9H-fluorene-2-carboxylate to react with sodium borohydride at room temperature in methanol to reduce the carbonyl group, and then allowing the thus obtained compound to react with diethylaminosulfur trifluoride at room temperature in methylene chloride. FAB-MS: 243 (M + H)⁺.

### Reference Example 10

Propyl spiro[1,3-dioxolane-2,9'-fluorene]-2'-carboxylate was obtained by allowing propyl 9-oxo-9H-fluorene-2-carboxylate to react with ethylene glycol and p-toluenesulfonic acid under heating in benzene. FAB-MS: 311 M + H)⁺.

### Reference Example 11

Propyl 9,9-dimethoxy-9H-fluorene-2-carboxylate was obtained by allowing propyl 9-oxo-9H-fluorene-2-carboxylate to react with methyl orthoformate and acetyl chloride in methanol at room temperature. FAB-MS: 313 (M + H)⁺.

### Reference Example 12

5'-Fluorospiro[1,3-dithiolane-2,9'-fluorene]-2'-carboxylate was obtained by allowing 5-fluoro-9-oxo-9H-fluorene-2-carboxylate to react with 1,2-ethanedithiol and boron trifluoride diethyl ether complex under heating in acetic acid. ESI-MS: 317 (M - H)⁻.

### Reference Example 13-a

Methyl (9EZ)-9-hydroxyimino-9H-fluorene-2-carboxylate was obtained by allowing methyl 9-oxo-9H-fluorene-2-carboxylate to react with hydroxylamine hydrochloride at room temperature in pyridine. FAB-MS: 254 (M + H)⁺.

### Reference Example 13-b

Methyl 9-acetylamino-9H-fluorene-2-carboxylate was obtained by treating methyl (9EZ)-9-hydroxyimino-9H-fluorene-2-carboxylate with 10% palladium-carbon and acetic anhydride in dioxane in an atmosphere of hydrogen gas. FAB-MS: 282 (M + H)⁺.

### Reference Example 14-a

1-Biphenyl-2-ylcyclopentanol was obtained by allowing 2-bromobiphenyl to react with n-butyl lithium (1.58 M, hexane solution) at -78°C in THF, and then adding THF solution of cyclopentanone and carrying out the reaction at room temperature. EI-MS: 238 (M)⁺.

### Reference Example 14-b

Spiro[cyclopentane-1,9'-fluorene] was obtained by allowing 1-biphenyl-2-ylcyclopentanol to undergo the reaction under heating in formic acid. EI-MS: 220 (M)⁺.

### Reference Example 14-c

2'-Bromospiro[cyclopentane-1,9'-fluorene] was obtained by bromination described in J. Am. Chem. Soc., 80, 4327 (1958) using spiro[cyclopentane-1,9'-fluorene]. EI-MS: 298 (M)⁺, 300 (M + 2)⁺.

### Reference Example 14-d

Spiro[cyclopentane-1,9'-fluorene]-2'-carbonitrile was obtained by allowing 2'-bromospiro[cyclopentane-1,9'-fluorene] to react with cupper cyanide under heating in DMF. ESI-MS: 246 (M + H)⁺.

### Reference Example 14-e

Spiro[cyclopentane-1,9'-fluorene]-2'-carboxylic acid was obtained by allowing ethanol solution of spiro[cyclopentane-1,9'-fluorene]-carbonitrile to react with 8 M potassium hydroxide aqueous solution under heating. ESI-MS: 263 (M + H)⁺.

### Reference Example 15

4-(4'-Methylbiphenyl2-yl)tetrahydro-2H-pyran-4-ol [EI-MS: 268 (M)⁺] was obtained by carrying out the reaction of Reference Example 14-a using 2-bromo-4'-methylbiphenyl and tetrahydro-4H-pyran-4-one as the starting materials, and then 2-methyl-2',3',5',6'-tetrahydrospiro[fluorene-9,4'-pyran] [ESI-MS: 251 (M + H)⁺] was produced in the same manner as in Reference Example 14-b. By allowing this to react with potassium permanganate under heating in pyridine-water, and allowing the thus obtained crude product to undergo the reaction under heating in methanol in the presence of sulfuric acid, methyl-2',3',5',6'-tetrahydrospiro[fluorene-9,4'-pyran]-2'-carboxylate was obtained. ESI-MS: 295 (M + H)⁺.

### Reference Example 16-a

9H-Fluorene-9,9-diyldimethylene dimethanesulfonate was obtained by allowing 9H-fluorene-9,9-diyldimethanol to react with methanesulfonyl chloride and triethylamine at room temperature in methylene chloride. EI-MS: 382 (M)⁺.

### Reference Example 16-b

9,9,-Bis(iodomethyl)-9H-fluorene was obtained by allowing 9H-fluorene-9,9-diyldimethylene dimethanesulfonate to react with sodium iodide under heating in hexamethylphosphoramide. By treating this with zinc under heating in ethanol, spiro[cyclopropane-1,9'-fluorene] was obtained (EI-MS: 192 (M)⁺). Thereafter, spiro[cyclopropane-1,9'-fluorene]-2'-carboxylic acid was produced in the same manner as in Reference Examples 14-c to 14-e. ESI-MS: 235 (M - H)⁻.

### Reference Example 17-a

9,9-Bis[2-(benzyloxy)ethyl]-2-bromo-9H-fluorene was obtained by allowing 2-bromo-9H-fluorene and [(2-chloroethoxy)methyl]benzene to undergo the reaction under heating in DMSO in the presence of potassium tert-butoxide. FAB-MS: 535 (M + Na)⁺, 537 (M + 2 + Na)⁺.

### Reference Example 17-b

Ethyl 9,9-bis[2-(benzyloxy)ethyl]-9H-fluorene-2-carboxylate was obtained by cyanation of the bromo group of 9,9-bis[2-(benzyloxy)ethyl]-2-bromo-9H-fluorene in the same manner as in Reference Example 14-d and subsequently converting into carboxyl group by the same hydrolysis reaction of Reference Example 14-e, and then carrying out esterification reaction in the same manner as in Reference Example 4-b using ethyl iodide. FAB-MS: 507 (M + H)⁺.

### Reference Example 17-c

Ethyl 9,9-bis(2-hydroxyethyl)-9H-fluorene-2-carboxylate was obtained by allowing ethyl 9,9-bis[2-(benzyloxy)ethyl]-9H-fluorene-2-carboxylate to react with palladium-carbon at room temperature in methanol in an atmosphere of hydrogen gas (FAB-MS: 327 (M + H)⁺) . The thus obtained compound was allowed to react with p-toluenesulfonyl chloride at room temperature in methylene chloride in the presence of triethylamine, and then the thus obtained compound was allowed to react with methylamine (40% methanol solution) under heating in dioxane in the presence of potassium carbonate to obtain methyl 1'-methylspiro[fluorene-9,4'-piperidine]-2-carboxylate. APCI: 308 (M + H)⁺.

### Reference Example 18-a

Methyl 9,9-bis(hydroxymethyl)-9H-fluorene-2-carboxylate was obtained by allowing methyl 9H-fluorene-2-carboxylate to react with paraformaldehyde at room temperature in DMSO in the presence of sodium ethoxide. FAB-MS: 284 (M)⁺.

### Reference Example 18-b

Methyl 9,9-bis({[tert-butyl(dimethyl)silyl]oxy} methyl)-9H-fluorene-2-carboxylate was obtained by allowing methyl 9,9-bis(hydroxymethyl)-9H-fluorene-2-carboxylate to react with tert-butyldimethylsilyl chloride at room temperature in pyridine. FAB-MS: 513 (M + H)⁺.

### Reference Example 19

9-Hydroxy-9-(tetrahydro-2H-pyran-4-yl)-9H-fluorene-2-carboxylic acid [FAB-MS: 309 (M - H)⁻] produced from 4-tetrahydro-2H-pyranyl magnesium chloride (prepared from 4-chlorotetrahydro-2H-pyran and magnesium) and 9-oxo-9H-fluorene-2-carboxylic acid in the same manner as in Reference Example 4-a was allowed to react with triethylsilane at room temperature in trifluoroacetic acid, thereby obtaining 9-(tetrahydro-2H-pyran-4-yl)-9H-fluorene-2-carboxylic acid. FAB-MS: 291 (M - H)⁻.

### Reference Example 20

9-(Tetrahydro-4H-pyran-4-ylidene)-9H-fluorene-2-carboxylic acid was obtained by allowing 9-hydroxy-9-(tetrahydro-2H-pyran-4-yl)-9H-fluorene-2-carboxylic acid to react with 6 M hydrochloric acid under heating in dioxane. FAB-MS: 294 (M)⁺.

### Reference Example 21-a

2-Fluoro-4'-methyl-6-nitrobiphenyl [FAB-MS: 232 (M + H)⁺] was obtained from 2-fluoro-6-nitrophenyl trifluoromethanesulfonate and 4-methylphenylboric acid by carrying out the reaction in the same manner as in Reference Example 1-a, and converted into (6-fluoro-4'-methylbiphenyl-2-yl)amine [EI-MS: 201 (M)⁺] by subjecting this nitro group to catalytic hydrogenation reduction, and then Sandmeyer reaction was carried out to obtain 2-bromo-6-fluoro-4'-methylbiphenyl. EI-MS: 266 (M)⁺, 268 (M)⁺.

### Reference Example 21-b

4-(6-Fluoro-4'-methylbiphenyl-2-yl)tetrahydro-2H-pyran-4-ol [EI-MS: 286 (M)⁺] was obtained by carrying out the reaction of 2-bromo-6-fluoro-4'-methylbiphenyl with tetrahydro-4H-pyran-4-one in the same manner as in Reference Example 14-a using t-butyl lithium (1.48 M, pentane solution), and then further reaction was carried out in the same manner as in Reference Example 14-b to obtain 5-fluoro-2-methyl-2',3',5',6'-tetrahydrospiro[fluorene-9,4'-pyran]. FAB-MS: 268 (M)⁺.

### Reference Example 21-c

5-Fluoro-2',3',5',6'-tetrahydrospiro[fluorene-9,4'-pyran]-2-carboaldehyde [FAB-MS: 283 (M + H)⁺] was obtained by allowing 5-fluoro-2-methyl-2',3',5',6'-tetrahydrospiro[fluorene-9,4'-pyran], N-bromosuccinimide and 2,2'-azobisisobutyronitrile to undergo the reaction under heating in carbon tetrachloride, and by allowing the thus obtained crude product to react with silver nitrate in acetone-water. By further allowing this compound to react with sodium perchlorate, sodium dihydrogenphosphate and 2-methyl-2-butene at room temperature in a mixed solvent of tert-butanol-acetonitrile-water, 5-fluoro-2',3',5',6'-tetrahydrospiro[fluorene-9,4'-pyran]-2-carboxylic acid was obtained. FAB-MS: 299 (M + H)⁺.

Compounds of Reference Examples 22 to 111 were produced in the same manner as in the above Reference Examples. Their structural formulae and physical properties are shown in the Tables 1 to 6 which are described later.

### Example 1

A 402 mg portion of CDI was added to 20 ml DMF solution of 400 mg 5-fluoro-9-oxo-9H-fluorene-2-carboxylic acid and stirred at 50°C for 1 hour. After cooling to room temperature, 743 mg of guanidine carbonate was added thereto and stirred overnight. After evaporation of the solvent, water was added thereto, and the thus precipitated solid was purified by a silica gel column chromatography (Chromatorex (registered trademark), methanol/chloroform) to obtain 434 mg of N-(diaminomethylene)-5-fluoro-9-oxo-9H-fluorene-2-carboxamide as yellow solid.

### Example 2

A 2.67 g portion of 1,1'-carbonyldiimidazole was added to 60 ml dimethylformamide (DMF) solution of 3.35 g 9-oxo-9H-fluorene-2-carboxylic acid and stirred at room temperature for 2.25 hours. This solution was added under ice-cooling to a solution which had been prepared by adding 3.00 g of sodium hydride to 20 ml DMF solution of 7.16 g guanidine hydrochloride and stirring at room temperature for 1.5 hours, and the mixture was stirred at room temperature for 1.5 hours. After evaporation of the solvent, water and ethyl acetate were added thereto, and the precipitated solid was washed with methanol to obtain 3.00 g of N-(diaminomethylene)-9-oxo-9H-fluorene-2-carboxamide as yellow solid.

### Example 3

A 110 mg portion of sodium borohydride was added to 10 ml methanol solution of 400 mg N-(diaminomethylene)-9-oxo-9H-fluorene-2-carboxamide and stirred at room temperature for 1 hour. After evaporation of the solvent, chloroform and 1 M sodium hydroxide aqueous solution were added thereto, and the precipitated solid was dissolved in 30 ml of ethanol, mixed with 0.2 ml of 4 M hydrogen chloride-ethyl acetate solution and stirred at room temperature for 1.5 hours. By collecting the thus formed solid by filtration, 380 mg of N-(diaminomethylene)-9-hydroxy-9H-fluorene-2-carboxamide hydrochloride was obtained as white solid.

### Example 4

A 1.0 g portion of sulfonyl chloride was added to 20 ml methylene chloride suspension of 480 mg N-(diaminomethylene)-9-hydroxy-9H-fluorene-2-carboxamide and stirred at room temperature for 30 minutes. After evaporation of the solvent, the residue was purified by a column chromatography (silica gel 60, methanol/chloroform) to obtain 155 mg of 9-chloro-N-(diaminomethylene)-9H-fluorene-2-carboxamide.

### Example 5

A 2 ml portion of 4 M hydrogen chloride-ethyl acetate solution was added to 10 ml methanol solution of 170 mg of tert-butyl-(2-{[(diaminomethylene)amino]carbonyl}-9H-fluoren-9-yl)carbamate produced in the same manner as in Example 1, and the mixture was stirred at 60°C for 20 minutes. By washing the thus obtained solid with hot ethanol, 83 mg of 9-amino-N-(diaminomethylene)-9H-fluorene-2-carboxamide dihydrochloride was obtained.

### Example 6

A 1 ml portion of 4 M hydrogen chloride-methanol solution was added to 5 ml methanol solution of 490.mg.N-(diaminomethylene)-9,9-bis({[tert-butyl(dimethyl)silyl]oxy}methyl)-9H-fluorene-2-carboxamide and stirred at room temperature for 2 hours. By evaporating the solvent and washing the residue with ethyl acetate, 250 mg of N-(diaminomethylene)-9,9-bis(hydroxymethyl)-9H-fluorene-2-carboxamide hydrochloride was obtained.

### Example 7

A 20 mg portion of hydroxylamine hydrochloride was added to 3 ml pyridine solution of 55 mg N-(diaminomethylene)-8-hydroxymethyl-9-oxo-9H-fluorene-2-carboxamide and stirred at room temperature for 10 hours. After evaporation of the solvent, water-ethanol was added thereto, and the precipitated solid was purified by Chromatorex (methanol/chloroform) to obtain 14 mg of (9EZ)-N-(diaminomethylene)-9-hydroxyimino-8-hydroxymethyl-9H-fluorene-2-carboxamide as white solid.

### Example 8

A 420 mg portion of (2R)-2-[(tert-butoxycarbonyl)amino]-3-methylbutanoic acid, 400 mg of 1-[3-(dimethylamino)propyl]-3-ethylcarbodiimide and 22 mg of 4-(N,N-dimethylamino)pyridine were added to 8 ml DMF solution of 470 mg N-(diaminomethylene)-9-hydroxy-9H-fluorene-2-carboxamide and stirred overnight at room temperature. After evaporation of the solvent, chloroform and saturated sodium bicarbonate aqueous solution were added thereto to remove the insoluble matter, and the organic layer was washed with saturated brine and dried with magnesium sulfate. After evaporation of the solvent, the residue was purified by a column chromatography (silica gel 60, methanol/chloroform). A 270 mg portion of the thus obtained compound was dissolved in 10 ml of ethanol, mixed with 2 ml of 4 M hydrogen chloride-ethyl acetate solution and stirred overnight at 40°C. After evaporation of the solvent, the residue was recrystallized from 2-propanol to obtain 30 mg of 9-hydroxy-N-[(2EZ,4S)-4-isopropyl-5-oxoimidazolin-2-ylidene-9H-fluorene-2-carboxamide hydrochloride.

### Example 9

A 2.99 ml portion of sodium methoxide-methanol solution (28%) was added to 8 ml DMF solution of 1.38 g guanidine hydrochloride and stirred at room temperature for 1 hour. A 4 ml portion of DMF solution of 350 mg ethyl 6-methyl-9-oxo-9H-fluorene-2-carboxylate was added to this solution and stirred at 100°C for 3 hours. This was spontaneously cooled to room temperature, the solvent was evaporated, and then methanol, water and 1 M sodium hydroxide aqueous solution were added thereto, and the thus precipitated solid was washed with 5 ml of methanol to obtain 215 mg of N-(diaminomethylene)-6-methyl-9-oxo-9H-fluorene-2-carboxamide as yellow solid.

Compounds of Examples 10 to 110 were produced in the same manner as in the above Examples. Their structural formulae and physical properties are shown in the Tables 7 to 18 which are described later. In addition, the compounds of Table 19 and Table 20, which are described later, may be easily produced in almost the same manner as in the above Examples or the methods described in the production methods, or by applying slight modifications obvious for those skilled in the art to these methods.

In this connection, the 6 compounds of Examples 56a and 56b, 60a and 60b, 78a and 78b were produced by carrying out resolution of respective compounds of Examples 56, 60 and 77 produced as racemic compounds, using chiral columns. The used columns and solvents used as the mobile phase are shown below.
Examples 56a and 56b
Used column: CHIRALPAK AD-H, mobile phase:
ethanol/diethylamine.
Examples 60a and 60b
Used column: CHIRALPAK OJ, mobile phase:
methanol/diethylamine.
Examples 78a and 78b
Used column: CHIRALPAK AD-H, mobile phase:
hexane/ethanol/triethylamine.

Symbols in the tables have the following meanings. Each numeral before the substituent group shows the substituted position.

Me, methyl, Et: ethyl, Bu: normal butyl, REx: Reference Example number, Ex: Example number, Cmp: compound number, RSyn and Syn: production methods (numerals indicate Reference Example number and Example number of compound produced in the same manner), Str: structure, Sal: salt (not described: free form; HCl: hydrochloride; numeral indicates molar ratio of the acid component, for example, 2 HCl means dihydrochloride), Dat: physicochemical properties(FAB: FAB-MS, ESI: ESI-MS, EI: EI-MS, NMR: δ values of typical peaks of nuclear magnetic resonance spectrum (DMSO-_{d6}, TMS internal standard)).

In this connection, the compound in which "*" was added to the substituent group in respective table indicates that it is one of the chiral compounds obtained by separation of optical isomers based on the asymmetry of carbon bonded to said substituent group. In addition, the following symbols shown in the tables show analytical conditions of high performance liquid chromatography. Proc. A: (column: CHIRALPAK AD-H [0.46 cm I.D. x 25 cm], mobile phase, methanol/diethylamine = 100/0.1, flow rate: 0.5 ml/min, temperature: 20°C, wavelength: 260 nM), Proc. B: (column: CHIRALPAK OJ [0.46 cm I.D. x 25 cm], mobile phase, ethanol/diethylamine = 100/0.1, flow rate: 0.3 ml/min, temperature: 40°C, wavelength: 264 nM), Proc. C: (column: CHIRALPAK AD-H [0.46 cm I.D. x 25 cm], mobile phase, hexane/ethanol/triethylamine = 50/50/0.05, flow rate: 1.0 ml/min, temperature: 25°C, wavelength: 257 nM).

**Table 1**

| | | | | |
|---|---|---|---|---|
| 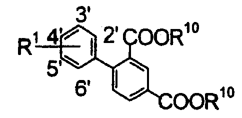 | | | | |

| REx | RSyn | R¹ | R¹⁰ | Dat |
|---|---|---|---|---|
| 22 | 1-a | 2'-F | -Et | ESI:317(M+H)⁺ |
| 23 | 1-a | 3'-F | -Et | FAB:317(M+H)⁺ |
| 24 | 1-a | 4'-F | -Et | FAB:317(M+H)⁺ |
| 25 | 1-a | 2'-Cl | -Et | FAB:333(M+H)⁺ |
| 26 | 1-a | 3'-Cl | -Et | FAB:333(M+H)⁺ |
| 27 | 1-a | 4'-Cl | -Et | FAB:333 (M+H)⁺ |
| 28 | 1-a | 3'-Me | -Et | FAB:313(M+H)⁺ |
| 29 | 1-a | 3'-OMe | -Et | FAB:328(M)⁺ |
| 30 | 1-a | 4'-OMe | -Et | FAB:329(M+H)⁺ |
| 31 | 1-a | 2'-Et | -Et | ESI:327(M+H)⁺ |
| 32 | 1-a | 2'-CF₃ | -Et | FAB:367 (M+H)⁺ |
| 33 | 1-a | 2'-F, 5'-Me | -Et | ESI:331 (M+H)⁺ |
| 34 | 1-a | 2 =Me, 5'-Me | -Et | ESI:327(M+H)⁺ |
| 35 | 1-b | 2'-F | -H | FAB:261 (M+H)⁺ |
| 36 | 1-b | 3'-F | -H | FAB:259(M-H)⁻ |
| 37 | 1-b | 4'-F | -H | FAB:261(M+H)⁺ |
| 38 | 1-b | 2'-Cl | -H | FAB:277 (M+H)⁺ |
| 39 | 1-b | 3'-Cl | -H | FAB:277 (M+H)⁺ |
| 40 | 1-b | 4'-Cl | -H | FAB:277 (M+H)⁺ |
| 41 | 1-b | 3'-Me | -H | FAB:257 (M+H)⁺ |
| 42 | 1-b | 3'-OMe | -H | FAB:271 (M-H)⁻ |
| 43 | 1-b | 4'-OMe | -H | FAB:273 (M+H)⁺ |
| 44 | 1-b | 2'-Et | -H | FAB:269 (M-H)⁻ |
| 45 | 1-b | 2'-CF₃ | -H | FAB:309 (M-H)⁻ |
| 46 | 1-b | 2'-F, 5'-Me | -H | ESI:273 (M-H)⁻ |
| 47 | 1-b | 2'-Me, 5'-Me | -H | FAB:269 (M-H)⁻ |

**Table 2**

| | | | | | |
|---|---|---|---|---|---|
| 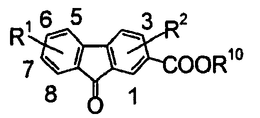 | | | | | |

| REx | RSyn | R¹ | R² | R¹⁰ | Dat |
|---|---|---|---|---|---|
| 48 | 1-c | 5-F | -H | -H | FAB:242(M)⁻ |
| 49 | 1-c | 7-F | -H | -H | FAB:243 (M+H)⁺ |
| 50 | 1-c | 8-F | -H | -H | FAB:243 (M+H)⁺ |
| 51 | 1-c | 5-Cl | -H | -H | FAB:259(M+H)⁺ |
| 52 | 1-c | 7-Cl | -H | -H | FAB:259 (M+H)⁺ |
| 53 | 1-c | 8-Me | -H | -H | FAB:239 (M+H)⁺ |
| 54 | 1-c | 5-Et | -H | -H | FAB:251 (M-H)⁻ |
| 55 | 1-c | 5-CF₃ | -H | -H | FAB:291(M-H)⁻ |
| 56 | 1-c | 7-OMe | -H | -H | FAB:255 (M+H)⁺ |
| 57 | 1-c | 5-F, 8-Me | -H | -H | ESI:255 (M-H)⁻ |
| 58 | 1-c | 5-Me, 8-Me | -H | -H | FAB:251 (M-H)⁻ |
| 59 | 1-c | -H | 1-Cl | -H | FAB:259(M+H)⁺ |
| 60 | 1-c | -H | 3-Cl | -H | FAB:259(M+H)⁺ |
| 61 | 2 | 5-F | -H | -Et | FAB:271 (M+H)⁺ |
| 62 | 2 | 8-F | -H | -Et | FAB:271 (M+H)⁺ |
| 63 | 2 | 5-Me | -H | -Me | FAB:253 (M+H)⁺ |
| 64 | 2 | 6-OMe | -H | -Et | FAB:283 (M+H)⁺ |
| 65 | 2 | 8-OMe | -H | -Et | FAB:283 (M+H)⁺ |
| 66 | 2 | -H | 1-Cl | -Et | FAB:287 (M+H)⁺ |
| 67 | 2 | -H | 3-Cl | -Et | FAB:287(M+H)⁺ |
| 68 | 4-b | 6-Cl | -H | -CH₂CH=CH₂ | FAB:299(M+H)⁺ |
| 69 | 4-b | 8-Cl | -H | -CH₂CH=CH₂ | FAB:299 (M+H)⁺ |
| 70 | 7-b | 5-CH₂NMe₂ | -H | -Me | FAB:296(M+H)⁺ |
| 71 | 8-a | 5-CH₂OAc | -H | -Me | FAB:311 (M+H)⁺ |
| 72 | 8-c | 5-H₂OMe | -H | -Me | FAB:283 (M+H)⁺ |

**Table 3**

| | | | | | | |
|---|---|---|---|---|---|---|
| 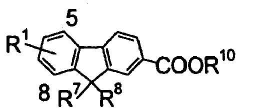 | | | | | | |

| REx | RSyn | R¹ | R⁷ | R⁸ | R¹⁰ | Dat |
|---|---|---|---|---|---|---|
| 73 | 4-a | -H | -Et | -OH | -H | FAB:253(M-H)⁻ |
| 74 | 4-a | -H | -Bu | -OH | -H | ESI:281(M-H)⁻ |
| 75 | 4-a | 5-F | -Me | -OH | -H | FAB:257(M-H)⁻ |
| 76 | 4-a | 5-Me | -Me | -OH | -H | EI:254(M)⁺ |
| 77 | 4-a | 5-Et | -Me | -OH | -H | FAB:267(M-H)⁻ |
| 78 | 4-a | 5-CF₃ | -Me | -OH | -H | FAB:307(M-H)⁻ |
| 79 | 4-a | 8-Me | -Me | -OH | -H | FAB:253(M-H)⁻ |
| 80 | 4-a | 5-F, 8-Me | -Me | -OH | -H | FAB:271(M-H)⁻ |
| 81 | 4-a | 5-Me, 8-Me | -Me | -OH | -H | FAB:267(M-H)⁻ |
| 82 | 4-b | -H | -Et | -OH | -Me | FAB:269(M+H)⁺ |
| 83 | 4-b | 5-F | -Me | -OH | -Me | EI:272(M)⁺ |
| 84 | 4-b | 8-Me | -Me | -OH | -Me | EI:268(M)⁺ |
| 85 | 4-b | 5-F, 8-Me | -Me | -OH | -Me | FAB:285(M-H)⁻ |
| 86 | 4c | -H | -Et | -OMe | -Me | FAB:283(M+H)⁺ |
| 87 | 4-c | 5-F | -Me | -OMe | -Me | FAB:287(M+H)⁺ |
| 88 | 4-c | 8-Me | -Me | -OMe | -Me | FAB:283(M+H)⁺ |
| 89 | 4-c | 5-F, 8-Me | -Me | -OMe | -Me | FAB:301 (M+H)⁺ |
| 90 | 5 | -H | -Me | -O(CH₂)₂OMe | -Me | EI:312(M)+ |
| 91 | 7-b | 5-F, 8-CH₂-NMe₂ | -Me | -OMe | -Me | FAB:344(M+H)⁺ |
| 92 | 7-b | 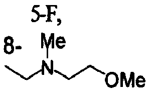 | -Me | -OMe | -Me | FAB:388(M+H)⁺ |
| 93 | 14-e | -H | -CH₂OMe | -CH₂OMe | H | ESI:297(M-H)⁻ |
| 94 | 14-e | -H | -(CH₂)₂OMe | -(CH₂)₂OMe | -H | ESI:325(M-H)⁻ |
| 95 | 14-e | -H | -(CH₂)₂OBn | -(CH₂)₂OBn | -H | ESI:477(M-H)⁻ |
| 96 | 1-b | -H | -F | -H | -H | FAB:227(M-H)⁻ |

**Table 4**

| | | | | | | |
|---|---|---|---|---|---|---|
| 97 | 1-b | -H | NHAc | -H | -H | FAB:268 (M+H)⁺ |
| 98 | 1-b | -H | -NHBOC | -H | -H | FAB:338(M-H)⁻ |
| 99 | 1-b | -H | -(CH₂)₃OH | -OH | -H | EI:284(M)⁺ |
| 100 | 1-b | -H | -CH₂OTBS | -CH₂OTBS | -H | FAB:499(M+H)⁺ |

**Table 5**

| | | | | | |
|---|---|---|---|---|---|
| 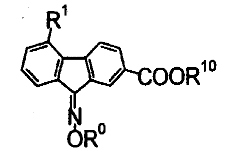 | | | | | |

| REx | RSyn | R⁰ | R¹ | R¹⁰ | Dat |
|---|---|---|---|---|---|
| 101 | 13-a | -H | -F | -H | FAB:286(M+H)⁺ |
| 102 | 13-a | -Me | -H | -Pr | FAB:296(M+H)⁺ |

**Table 6**

| | | | | | |
|---|---|---|---|---|---|
| 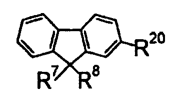 | | | | | |

| REx | RSyn | R⁷ | R⁸ | R²⁰ | Dat |
|---|---|---|---|---|---|
| 103 | 5 | -CH₂OMe | -CH₂OMe | -H | ESI:255(M+H)⁺ |
| 104 | 14-b | -(CH₂)₂O(CH₂)₂- | | -Me | FAB:251 (M+H)⁺ |
| 105 | 14-c | -CH₂OMe | -CH₂OMe | -Br | ESI:332(M+H)⁺, 334(M+H+2)⁺ |
| 106 | 14-c | -(CH₂)₂OMe | -(CH₂)₂OMe | -Br | FAB:361(M+H)⁺,363 (M+H+2)⁺ |
| 107 | 14-c | -(CH₂)₂- | | -Br | EI:270(M)⁺, 272(M+2)⁺ |
| 108 | 14-d | -CH₂OMe | -CH₂OMe | -CN | ESI:280(M+H)⁺ |
| 109 | 14-d | -(CH₂)₂OMe | -(CH₂)₂OMe | -CN | ESI:308 (M+H)⁺ |
| 110 | 14-d | -(CH₂)₂- | | -CN | FAB:218 (M+H)⁺ |
| 111 | 17-a | -(CH₂)₂OMe | -(CH₂)₂OMe | -H | FAB:283 (M+H)⁺ |

**Table 7**

| | | | |
|---|---|---|---|
| 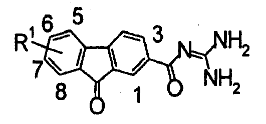 | | | |

| Ex | Syn | R¹ | Dat |
|---|---|---|---|
| 1 | 1 | 5-F | NMR:7.45-7.55(3H,m),7.73(1H,d,J=73Hz),836(1H,s).; FAB:284(M+H)⁺ |
| 2 | 2 | -H | NMR:7.42(1H,t,*J*=8.3Hz),7.62-7.67(2H,m),831(1H,5); FAB:266(M+H)⁺ |
| 9 | 9 | 6-Me | NMR:2.42(3H,s),7.22(1H,d,*J*=7.3Hz),8.29(1H,s).;FAB: 280(M+H)⁺ |
| 10 | 1 | 7-F | NMR:7.46-7.51(2H,m)828(1H,dd,*J*=7.8,1.5Hz),8.31(1H, d,*J*=1.2Hz.;FAB:284(M+H)⁺ |
| 11 | 1 | 8-F | NMR:7.21(1H,ddd,*J*=9.8,83,25Hz)7.70(1H,dd,*J*=83, 53Hz),831(1H,s).;FAB:284(M+H)⁺ |
| 12 | 2 | 1-Cl | NMR:7.43(1H,t,*J*=6.8Hz),7.62-7.69(3H,m),7.75(1H,d,*J*= 7.8Hz).;FAB:300(M+H)⁺ |
| 13 | 2 | 3-Cl | NMR:7.44(1H,t,*J*=7.3Hz),7.79(1H,s),7.94(1H,s).;FAB: 300(M+H)⁺ |
| 14 | 2 | 5-Cl | NMR:7.44(1H,t,*J*=7.8Hz),8.18(1H,d,*J*=7.8Hz),837(1H, d,*J*=1.0Hz).;FAB:300(M+H)⁺ |
| 15 | 2 | 7-Cl | NMR:7.64(1H,d,*J*=1.9Hz),7.84-7.89(2H,m),8.32(1H,s).; FAB:300(M+H)⁺ |
| 16 | 2 | 5-Me | NNR:261(3H,s),7.31(1H,t,*J*=7.6Hz),8.33(1H,d,*J*=0.9 Hz).;FAB:280(M+H)⁺ |
| 17 | 2 | 5-Et | NMR:1.29(3H,t,*J*=73Hz),736(1H,t,*J*=73Hz),8.33(1H, d,*J*=1.4Hz).;FAB:294(M+H)⁺ |
| 18 | 2 | 7-OMe | NMR:3.85(3H,s),7.69(1H,d,*J*=7.8Hz),8.26(1H,s).;FAB: 296(M+H)⁺ |
| 19 | 9 | 6-Cl | NMR:7.64(1H,d,*J*=7.3Hz),8.02(1H,d,*J*=1.5Hz),8.33(1H, s).;FAB:300(M+H)⁺ |
| 20 | 9 | 8-Cl | NMR:7.40(1H,d,*J*=7.8Hz),7.63(1H,t,*J*=7.8Hz),8.32(1H, s).;FAB:300(M+H)⁺ |
| 21 | 9 | 8-Me | NMR:2.56(3H,s),7.19(1H,d,*J*=7.8Hz),8.27(1H,s).;FAB: 280(M+H)⁺ |
| 22 | 9 | 6-OMe | NMR:3.92(3H,s),6.90(1H,dd,*J*=8.3Hz,20Hz),8.26-8.27 (2H,m).;FAB:296(M+H)⁺ |
| 23 | 9 | 8-OMe | NMR:3.91(3H,s),7.61(1H,t,*J*=8.1Hz),8.26(1H,s).;FAB: 296(M+H)⁺ |
| 24 | 9 | 5-CH₂NMe₂ | NMR:2.45(6H,s),3.66(2H,s),8.32(1H,d,*J*=1.0Hz).;FAB: 323(M+H)⁺ |

**Table 8**

| | | | |
|---|---|---|---|
| 25 | 9 | 8-CH₂NMe₂ | NMR:2.21(6H,s),3.85(2H,s),8.25(1H,d,*J*=1.4Hz).;FAB: 323(M+H)⁺ |
| 26 | 9 | 5-CH₂OH | NMR:4.84(2H,d,*J*=4.4Hz),7.41(1H,t,*J*=7.4Hz),8.32(1H, d,*J=*1.4Hz).;FAB.:296(M+H)⁺ |
| 27 | 9 | 8-CH₂OH | NMR:4.94(2H,d,*J*=5.9Hz),7.55(1H,dd,*J*=7.8,1.0Hz), 8.25(1H,s).;FAB:295(M)⁺ |
| 28 | 9 | 5-CH₂OMe | NMR:3.42(3H,s),4.75(2H,s),834(1H,d,*J*=1.4Hz).;FAB: 310(M+H)⁺ |
| 29 | 9 | 8-CH₂OMe | NMR:3.40(3H,s),4.86(2H,s),826(1H,s).;FAB:310 (M+H)⁺ |

**Table 9**

| | | | | |
|---|---|---|---|---|
| 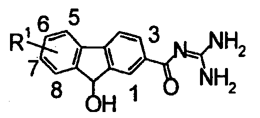 | | | | |

| Ex | Syn | R¹ | Sal | Dat |
|---|---|---|---|---|
| 3 | 3 | -H | HCl | NMR:5.59(1H,s),7.40-7.49(2H,m),8.23-8.28(2H);m).; FAB:268(M+H)⁺ |
| 30 | 3 | 5-F | HCl | NMR:5.67(1H,s),730(1H,t,*J*=83Hz),8.28(1H,s).; FAB:286(M+H)⁺ |
| 31 | 3 | 7-F | | NMR:5.49(1H,d,*J*=7.3Hz),7.18-7.26(1H,m),8.32(1H, s).;FAB:286(M+H)⁺ |
| 32 | 3 | 8-F | | NMR:5.48(1H,d,*J*=7.8Hz),7.14(1H,ddd,*J*=9.8,8.3, 2.4Hz),833(1H,s).;FAB:286(M+H)⁺ |
| 33 | 3 | 1-Cl | HCl | NMR:5.67(1H,s),7.43-7.49(2H,m),7.65(1H,dd,*J*=6.3, 2.0Hz).;FAB:302(M+H)⁺ |
| 34 | 3 | 3-Cl | HCl | NMR:5.56(1H,s),7.42-7.48(2H,m),8.13(1H,s).;FAB: 302(M+H)⁺ |
| 35 | 3 | 5-Cl | HCl | NMR:5.63(1H,s),7.65(1H,d,*J*=6.8Hz),829-831(2H, m).;FAB:302(M+H)⁺ |
| 36 | 3 | 6-Cl | HCl | NMR:5.59(1Hs),7.47(1H,dd,*J*=8.1,1.7Hz),8.08-8.10 (2H,m),.;FAB:302(M+H)⁺ |
| 37 | 3 | 7-Cl | HCl | NMR:5.59(1H,d,*J*=7.3Hz),7.65(1H,s),8.27(1H,s).; FAB:302(M+H)⁺ |
| 38 | 3 | 8-Cl | HCl | NMR:5.70(1H,s),7.49(1H,t,*J*=7.8Hz),8.25(1H,s).; FAB:302(M+H)⁺ |
| 39 | 3 | 5-Me | HCl | NMR:2.67(3H,s),5.54(1H,s),8.27(1H.s).;FAB:282 (M+H)⁺ |

**Table 10**

| | | | | |
|---|---|---|---|---|
| 40 | 3 | 6-Me | HCl | NMR:2.41(3H,s),5.54(1H,s),822(1H,s).;FAB:282 (M+H)⁺ |
| 41 | 3 | 8-Me | HCl | NMR:2.50(3H,s),5.64(1H,s),8.20(1H,s).;FAB:282 (M+H)⁺ |
| 42 | 3 | 5-Et | | NMR:1.28(3H,t,*J*=73Hz),5.42(1H,d,*J*=73Hz),8.34 (1H,s).;FAB:296(M+H)⁺ |
| 43 | 3 | 6-OMe | HCl | NMR:3.85(3H,s),5.52(1Hs),8.21(1H,s).;FAB:298 (M+H)⁺ |
| 44 | 3 | 7-OMe | HCl | NMR:3.85(3H,s),5.53(1H,s),8.20(1H,s).;FAB:298 (M+H) |
| 45 | 3 | 8-OMe | HCl | NMR:3.89(3H,s),5.66(2H,brs),8.20(1H,s).;FAB:298 (M+H)⁺ |
| 46 | 3 | 5-CH₂NMe₂ | 2HCl | NMR:2.85(6H, s),5.58 (1H,s),8.32(1H,s).;FAB:325 (M+H)⁺ |
| 47 | 3 | 8-CH₂NMe₂ FAB: | 2HCl | NMR:2.79 and 2.85(6H,s and s),5.99(1H,s),8.33(1H,s).; 325(M+H)⁺ |
| 48 | 3 | 5-CH₂OH | HCl | NMR:4.89(2H,s),5.56(1H,s),8.24(1H,s).;FAB:298 (M+H)⁺ |
| 49 | 3 | 8-CH₂OH | HCl | NMR:4.84(2H,s),5.69(1H,s),7.44-7.50(2H,m).;FAB: 298 (M+H)⁺ |
| 50 | 3 | 5-CH₂OMe | HCl | NMR:3.39(3H,s),5.57(1H,s),8.23(1H,s).;FAB:312 (M+H)⁺ |
| 51 | 3 | 8.CH₂OMe | HCl | NMR:3.39(3H,s),5.69(1H,s),8.24(1H,s).;FAB:312 (M+H)⁺ |

**Table 11**

| | | | | | |
|---|---|---|---|---|---|
| 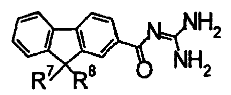 | | | | | |

| Ex | Syn | R⁷ | R⁸ | Sal | Dat |
|---|---|---|---|---|---|
| 4 | 4 | -H | -Cl | | NMR : 6.26 (1H,s),7.40-7.45 (1H, m), 8.36 (1H,s).;FAB:286(M+H)⁺ |
| 5 | 5 | -H | -NH₂ | 2HCl | NMR:5.54(1H,hs),754(1Hdt,*J*)=73,1.0 Hz),8.61(1H,s).;FAB:267(M+H)⁺ |
| 6 | 6 | -CH₂OH | -CH₂OH | HCl | NMR:3.78(4H,s), 738-7.46 (2H,m), 8.34 (1H, d, J=0.9 Hz). ; FAB : 312 (M+H) |

**Table 12**

| | | | | | |
|---|---|---|---|---|---|
| 52 | 2 | -H | -H | | NMR:3.96(2H,s),731-7.42(2H,m),8.31 (1H,s).;FAB:252(M+H)⁺ |
| 53 | 2 | -H | -Me | HCl | NMR:1.54(3H,d,*J*=7.3Hz),4.07(1H,q,*J* =7.3Hz),8.49(1Hs).;FAB:266(M+H)⁺ |
| 54 | 2 | -H | -F | HCl | NMR:6.60(1H,d,*J*=52.7Hz),7.49(1H,t,*J*=7.8Hz),8.36(1H,s).;FAB:270(M+H)⁺ |
| 55 | 2 | -H | -NHCOCH₃ | HCl | NMR:1.98(3H,s),6.11(1H,d,*J*=73Hz), 8.14(1H,s).;FAB:309(M+H)⁺ |
| 56 | 2 | -OH | -Me | HCl | NMR:1.64(3H,s),7.39-7.43(2H,m),8.26 (1H,d,*J*=1.4Hz).;FAB:282(M+H)⁺ |
| 56a | | -OH* | Me* | HCl | RT:7.39,Proc.A; FAB:282(M+H)⁺ |
| 56b | | -OH* | -Me* | HCl | RT:11.98,Proc.A; FAB:282(M+H)⁺ |
| 57 | 1 | -OH | -Et | HCl | NMR:0.42(3H,t,*J*=7.3Hz),2.04-2.18(2H, m),8.19(1H,d,*J*=1.0Hz).;FAB:296 (M+H)⁺ |
| 58 | 2 | -OH | -Bu | HCl | NMR:0.65-0.75(5H,m),739-7.44(2H,m),8.18(1H,brs);FAB:324(M+H)⁺ |
| 59 | 2 | -S(CH₂)₂S- | | HCl | NMR:3.83-3.90(4H,m),7.46-7.50(2H,m), 8.57(3H,m);FAB:342(M+H)⁺ |
| 60 | 9 | -O(CH₂)₃₋ | | HCl | NMR:2.25-244(4H,m),739-7.46(2H,m), 8.37(1H,d,*J*=15Hz).;FAB:308(M+H)⁺ |
| 60a | | -O(CH₂)₃-* | | HCl | 1434,Proc.B; FAB:308(M+H)⁺ |
| 60b | | -O(CH₂)₃-* | | HCl | RT:18.66,Proc.B; FAB:308(M+H)⁺ |
| 61 | 1 | -CH₂OMe | -CH₂OMe | HCl | NMR:3.17(6H,s),3.45(4Hs),8.39(1H,d,*J* =1.0Hz).;FAB:340(M+H)⁺ |
| 62 | 1 | -(CH₂)₂OMe | -(CH₂)₂OMe | HCl | NMR:233-248(4H,m),2.87(6H,s)8.56 (1H,s).;FAB:368(M+H)⁺ |
| 63 | 1 | -(CH₂)₂- | | HCl | NMR:1.81-1.86(2H,m),727-731(1H,m), 820(1H,d,*J*=1.4Hz).;FAB:278(M+H)⁺ |

**Table 13**

| | | | | | |
|---|---|---|---|---|---|
| 64 | 1 | -(CH₂)₄- | | HCl | NMR:1.94-2.02(2H,m),7.38-7.45(2H,m), 8.40(1H,s),.;FAB:306(M+H)⁺ |
| 65 | 1 | -OH | 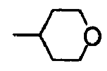 | HCl | NMR:1.37(2H,d,*J*=1.7Hz),7.40-7.46(2H, m),8.12(1H,s).;FAB:352(M+H)⁺ |
| 66 | 1 | -H | 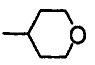 | HCl | NMR:4.12(1H,d,*J*=3.0Hz),7.43-7.48(2H, m),8.30(1H,s).;FAB:335(M+H)⁺ |
| 67 | 1 | 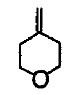 | | HCl | NMR:321-3.24(2H,m),7.43-7.45(2H,m), 8.57(1H,s).;FAB:334(M+H)⁺ |
| 68 | 9 | -OH | -OCH₂OMe | | NMR:1.64(3H,s),3.04(3H,s),8.24(1H,d,*J* =1.5Hz).;FAB:326(M+H)⁺ |
| 69 | 9 | -OH | -O(CH₂)₂OMe | | NMR:1.67(3H,s),3.13(3H,s),8.22-8.24 (3H,m).;FAB:340(M+H)⁺ |
| 70 | 9 | -OH | -O(CH₂)₃OH | HCl | NMR:0.87-0.94(2H,m),3.19(2H,t,*J*=6.6 Hz),8.17(1H,s).;FAB:326(M+H)⁺ |
| 71 | 9 | -OMe | -Me | HCl | NMR:1.67(3H,s),2.65(3H,s),8.30(1H,d,*J* =1.5Hz).;FAB:296(M+H)⁺ |
| 72 | 9 | -OMe | -Et | HCl | NMR:0.43(3H,t,*J*=7.6Hz),2.68(3H,s), 8.17(1H,d,*J*=1.5Hz).;FAB:310(M+H)⁺ |
| 73 | 9 | -OMe | -OMe | | NMR:3.29(6H,s),7.36(1H,dt,*J*=0.9,7.6 Hz),8.24(1H,s).;FAB:312(M+H)⁺ |
| 74 | 9 | -O(CH₂)₂O- | | | NMR:4.33-4.42(4H,m),7.32(1H,dt,*J*=1.0, 7.3Hz),8.21(1H,s).;FAB:310(M+H)⁺ |
| 75 | 9 | -(CH₂)₂O(CH₂)₂- | | HCl | NMR:1.76(2H,dt*,J=*13.7Hz,4.9Hz),4.06-4.11(4H,m),8.52(1H,s).;FAB:322 (M+H)⁺ |
| 76 | 9 | -(CH₂)₂NMe(CH₂)₂- | | 2HCl | NMR:1.63-1.71(2H,m),2.54(3H,s),8.92 (1H,s).;ESI:335(M+H)⁺ |

**Table 14**

| | | | | | | |
|---|---|---|---|---|---|---|
| 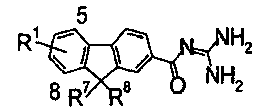 | | | | | | |

| Ex | Syn | R¹ | R⁷ | R⁸ | Sal | Dat |
|---|---|---|---|---|---|---|
| 77 | 1 | 5-F | -OH | Me | HCl | NMR: 1.69 (3H, s), 725-730 (1H, m), 8.29(1H,d,J=1.5Hz).;FAB:300 (M+H)⁺ |
| 78a | | 5-F | -OH* | -Me* | HCl | RT:3.97,Proc.C; |
| | | | | | | FAB:300(M+H)⁺ |
| 78b | | 5-F | -OH* | -Me* | HCl | RT:5.78,Proc.C; |
| | | | | | | FAB:300(M+H)⁺ |
| 79 | 1 | 5-Me | -OH | -Me | HCl | NMR:1.62(3H,s),2.66(3H,s),8.28(1H,s).;FAB:296(M+H)⁺ |
| 80 | 1 | 5-Et | -OH | -Me | HCl | NMR:129(3H,t,*J*=7.3Hz),1.61(3H,s),8.26(1H,d,*J*=1.9Hz).;FAB:310(M+H)⁺ |
| 81 | 1 | 5-CF₃ | -OH | -Me | HCl | NMR:1.67(3H,s),7.65(1H,t,*J*=7.8Hz),8.37(1H,d,*J*=1.4Hz).;FAB:350(M+H)⁺ |
| 82 | 1 | 8-Me | -OH | -Me | HCl | NMR:1.69(3H,s),2.55(3H,s),8.23(1H,d,*J*=1.5Hz).;FAB:296(M+H)⁺ |
| 83 | 1 | 5-Me,8-Me | -OH | -Me | HCl | NMR:1.67(3H,s),2.53(3H,s),2.67(3Hs);FAB:310(M+H)⁺ |
| 84 | 2 | 5-F,8-Me | -OH | -Me | HCl | NMR:1.70(3H,s),2.53(3H,s),8.29(1H,d,*J*=1.5Hz).;FAB:314(M+H)⁺ |
| 85 | 2 | 5-F | -S(CH₂)₂S | | HCl | NMR:3.87(1H,dt,*J=*11.0*,*3.9Hz),3.89(1H,dt,*J*=11.0,5.8Hz),8.64(1H,d,*J*=15Hz).;FAB:360(M+H)⁺ |
| 86 | 1 | 5-F | -(CH₂)₂O(CH₂)₂- | | HCl | NMR:1.74-1.80(2H,m),732(1H,t,*J*=8.3Hz),8.54(1H,s).;FAB:340(M+H)⁺ |
| 87 | 9 | 5-F | -OMe | -Me | HCl | NMR:1.69(3H,s),2.67(3H,s),8.32(1H,d,*J*=1.5Hz).;FAB:314(M+H)⁺ |
| 88 | 9 | 8-Me | -OMe | -Me | HCl | NMR:1.72(3H,s),268(3H,s),8.22(1H,d,*J*=1.5Hz).;FAB:310(M+H)⁺ |

**Table 15**

| | | | | | | |
|---|---|---|---|---|---|---|
| 89 | 9 | 5-F, 8-CH₂NMe₂ | -OMe | -Me | 2HCl | NMR:1.81(3H,s),2.76(3H,s),8.42(1H,s).;FAB:371(M+H)⁺ |
| 90 | 9 | 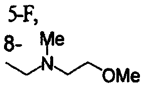 | -OMe | -Me | 2HCl | NMR:1.81and1.82(3H,sands),2.77 and2.78(3H,sands),184and2.85 (3H,sands).;FAB:415(M+H)⁺ |

**Table 16**

| | | | | | |
|---|---|---|---|---|---|
| 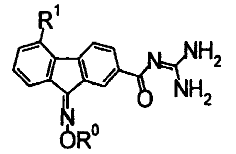 | | | | | |

| Ex | Syn | R⁰ | R¹ | Sal | Dat |
|---|---|---|---|---|---|
| 7 | 7 | -H | -CH₂OH | HCl | NMR:4.91and4.93(2H,sands),7.45-7.56(2H, m),7.63-7.74(2H,m).;FAB:311(M+H)⁺ |
| 91 | 9 | -H | -H | HCl | NMR:7.51(1H,dt,*J*=7.3,1.0Hz),7.59(1H,dt,*J*= 73,1.0Hz),836(1H,s).;FAB:281(M+H)⁺ |
| 92 | 9 | -Me | -H | HCl | NMR:4.24and425(3H,sands),7.44-7.65(2H, m),8.22-8.36(2H,mHz).;FAB:295(M+H)⁺ |
| 93 | 9 | -H | -F | | NMR:729-7.61(2H,m),7.78-7.84(1H,m),8.18-8.29(2H,m).;FAB:299(M+H)⁺ |
| 94 | 7 | -H | -CH₂NMe₂ | 2HCl | NMR.:2.87(6H,s),4.83(2H,s),7.44-7.65(1H,m).; FAB:338(M+H)⁺ |
| 95 | 7 | -H | -CH₂OMe | HCl | NMR:3.41and3.42(3H,sands),4.82and4.84 (2H,sands),7.43-7.60(2H,m).;FAB:325(M+H)⁺ |

**Table 17**

| | | | | | |
|---|---|---|---|---|---|
| 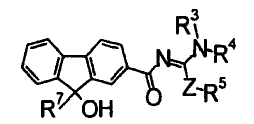 | | | | | |

| Ex | Syn | R⁷ | 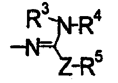 | | |
|---|---|---|---|---|---|
| 8 | 8 | H | 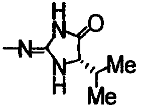 | | NMR:0.94-0.99(6H,m),420(1H,d,*J*=4.0 Hz),5.54(1H,s);FAB:350(M+H⁺) |
| 96 | 2 | Me | 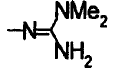 | HCl | NMR:1.63(3H,s),3.18(6Hs),8.16(1H,s); FAB:310(M+H)⁺ |
| 97 | 2 | Me | 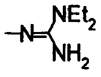 | HCl | NMR:121(6H,t,*J*=6.8Hz),1.63(3H,s), 8.16(1H,s);FAB:338(M+H)⁺ |
| 98 | 2 | Me | 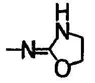 | | NMR:1.59(3H,s),3.80(2H,t,*J*=8.8Hz) 824(1H,brs);FAB:309(M+H)⁺ |
| 99 | 2 | Me | 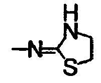 | HCl | NMR:1.61(3H,s),3.42(2H,t,*J*=8.8Hz), 8.27(1H,brs).;FAB:325(M+H)⁺ |
| 100 | 2 | Me | 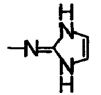 | | NMR:1.62(3H,s),6.83(2H,s),8.26(1H,d,*J* =0.8Hz).;FAB:306(M+H)⁺ |
| 101 | 2 | Me | 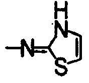 | | NMR:1.64(3H,s),7.29(1H,d,*J*=3.6Hz), 831(1H,d,*J*=1.6Hz).;FAB:323(M+H)⁺ |
| 102 | 2 | Me | 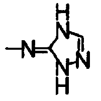 | | NMR:1.61(3H,s),7.91(1H,d,*J*=7.6Hz), 823(1H,d,*J*=1.6Hz).;FAB:307(M+H)⁺ |
| 103 | 2 | Me | 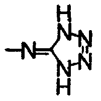 | | NMR:1.64(3H,s),7.94(1H,d,J=7.8Hz), 8.23(1H,d,*J*=1.6Hz).;FAB:308(M+H)⁺ |
| 104 | 2 | Me | -NH₂ | | NMR:1.60(3H,s),7.90(1H,dd,*J*=8.0,1.6 Hz),8.07(1H,d,*J*=1.2Hz).;FAB:240 (M+H)⁺ |

**Table 18**

| Ex | Syn | Str | Sal | Dot |
|---|---|---|---|---|
| 105 | 2 | 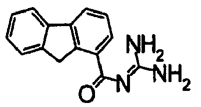 | HCl | NMR:4.26(2H,s),736-7.46(2H, m),8.25(1H,d,*J*=7.8Hz).;FAB: 252(M+H)+ |
| 106 | 2 | 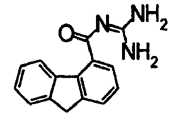 | HCl | NMR:4.01(2H,s),7.36-7.44(2H, m),7.83-7.91(1H,m).;FAB:252 (M+H)+ |
| 107 | 2 | 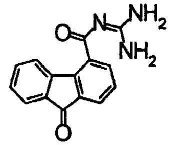 | | NMR:7.33-7.39(2H,m),7.75(1H, dd,*J*=8.0,12Hz),8.04(1H,d,*J*= 7.6Hz).;FAB:266(M+H)+ |
| 108 | 2 | 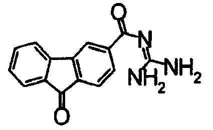 | HCl | NMR:7.47(1H,t*,J=*7.4Hz),7.68-7.73(2H,m),8.67(1H,s).;FAB:266 (M+H)+ |
| 109 | 3 | 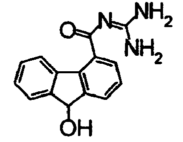 | HCl | NMR:5.51(1H,s),735-7.44(2H, m),7.83(1H,d,*J*=7.1Hz).;FAB: 268(M+H)+ |
| 110 | 3 | 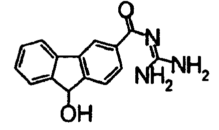 | HCl | NMR:5.59(1H,s),739(1H,t,*J*= 7.4Hz),8.74(1H,s).;FAB:268 (M+H)⁺ |

**Table 19**

| | | | | | |
|---|---|---|---|---|---|
| 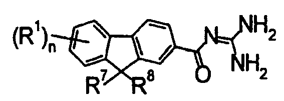 | | | | | |

| Cmp | 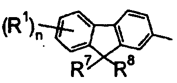 | Cmp | 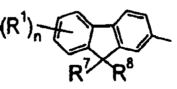 | Cmp | 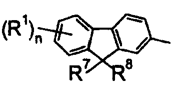 |
|---|---|---|---|---|---|
| 1 | 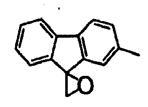 | 8 | 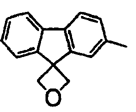 | 15 | 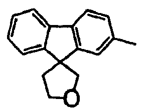 |
| 2 | 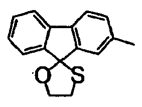 | 9 | 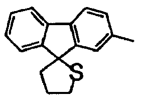 | 16 | 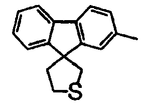 |
| 3 | 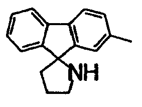 | 10 | 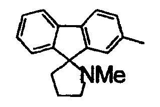 | 17 | 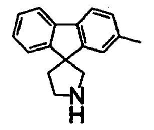 |
| 4 | 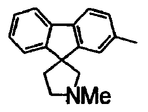 | 11 | 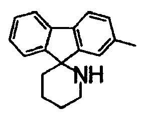 | 18 | 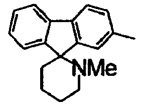 |
| 5 | 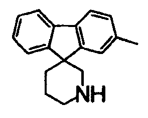 | 12 | 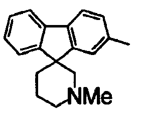 | 19 | 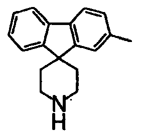 |
| 6 | 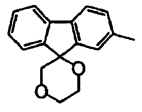 | 13 | 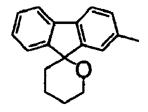 | 20 | 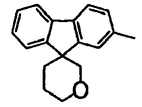 |
| 7 | 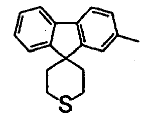 | 14 | 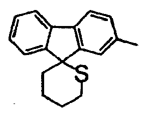 | 21 | 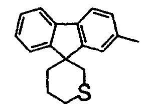 |

**Table 20**

| | | | | | |
|---|---|---|---|---|---|
| 22 | 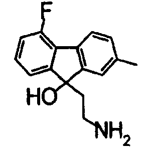 | 29 | 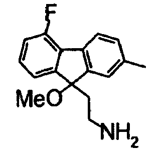 | 36 | 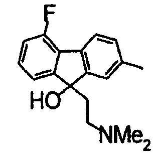 |
| 23 | 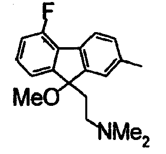 | 30 | 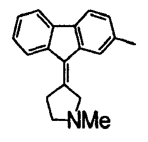 | 37 | 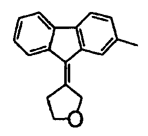 |
| 24 | 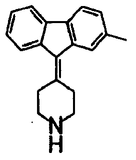 | 31 | 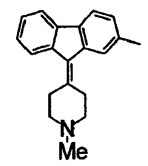 | 38 | 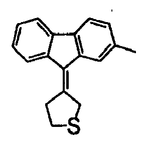 |
| 25 | 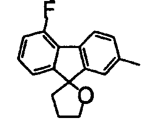 | 32 | 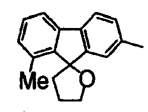 | 39 | 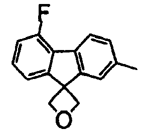 |
| 26 | 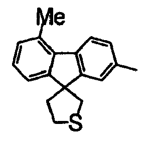 | 33 | 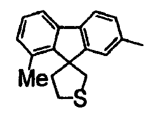 | 40 | 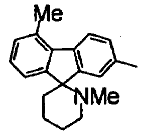 |
| 27 | 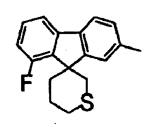 | 34 | 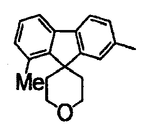 | 41 | 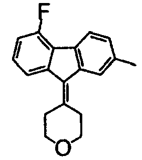 |
| 28 | 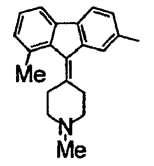 | 35 | 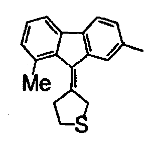 | 42 | 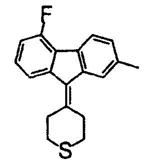 |

## Claims

1. A fluorene derivative represented by the following general formula (I) or a pharmaceutically acceptable salt thereof, 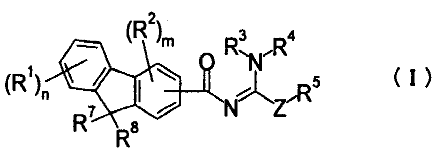 (symbols in the formula represent the following meanings,
R¹ and R²: the same or different from each other and each represents -R⁰, a lower alkenyl, a lower alkynyl, a halogen,
-OH, -O-R⁰ , -O-CO-R⁰, -NH₂, -NR⁶-R⁰ , -CN, -NO₂, -CHO, -CONH₂,
-CO-NR⁶-R⁰, -CO₂H, -CO₂-R⁰, -CO-R⁰, -NR-CO-R⁰, -NR⁶-CO₂-R⁰,
-O-CO-NR⁶-R⁰, -SH, -S(O)ₚ-R⁰, -S(O)₂-NH₂, O-S(O)₂-NR⁶-R⁰ ,
-NR⁶-S(O)₂-R⁰, -R⁰⁰-O-CO-R⁰, -R⁰⁰-NR⁶-R⁰, -R⁰⁰-CN, -R⁰⁰-CONH₂,
-R⁰⁰-CO-NR⁶-R⁰, -R⁰⁰-CO₂H, -R⁰⁰-CO₂-R⁰, -R⁰⁰-CO-R⁰,
-R⁰⁰-NR⁶-CO-R⁰, -R⁰⁰-NR⁶-CO₂-R⁰, -R⁰⁰-O-CO-NR⁶-R⁰, a cycloalkyl or a nitrogen-containing saturated hetero ring, wherein said nitrogen-containing saturated hetero ring may be substituted with 1 or 2 substituent groups selected from the group consisting of a lower alkyl, -OH, -O-R⁰, -NH₂, -NR⁶-R⁰ and oxo (=O) ;
R⁰: the same or different from one another and each represents a lower alkyl which may be substituted with one or more substituent groups selected from the group consisting of -OH, -O-C₁₋₄alkyl, -NH₂, -NR⁶-C₁₋₄ alkyl and a halogen;
R⁶: the same or different from one another and each represents a lower alkyl or H;
R⁰⁰: the same or different from one another and each represents a lower alkylene;
p: 0, 1 or 2;
n: 0, 1 or 2;
m: 0 or 1;
R⁷ and R⁸: the same or different from each other and each represents -H, -R⁰, a halogen, -OR, -O-R⁰, -NH₂, -NR⁶-R⁰ ,
-NR⁶-CO-R⁰, -O-R⁰⁰-OH, -O-R⁰⁰-O-R⁰, a cycloalkyl or an oxygen-containing saturated hetero ring, or R⁷ and R⁸ may together form a group selected from the group consisting of oxo (=0), =N-OH, =N-OR⁰ and tetrahydropyranylidene, or R⁷ and R⁸ may together form a lower alkylene which may be interrupted by 1 or 2 divalent groups selected from the class consisting of
-O-, -S(O)ₚ-, -NR⁶- and -CONR⁶-, and may form a 3- to 8-membered ring together with the C atom to which they are linked;
Z: -NH-;
R³: -H or R⁰; and
R⁴ and R⁵: the same or different from each other and each represents -H, -R⁰, -CO₂-R⁰, or -CO-R⁰, or R⁴ and R⁵ may together form a divalent group and may form a 5-membered hetero ring together with the -N-C-Z- group to which R⁴ and
R⁵ are linked, wherein Z may be -O- or S-, and said 5-membered ring may be substituted with 1 or 2 substituent groups selected from a lower alkyl, -OH, -O-R⁰, -NH₂, -NR⁶-R⁰ and oxo (=O)).

2. The fluorene derivative or pharmaceutically acceptable salt thereof described in claim 1, wherein R³ is -H or R⁰, and R⁴ and R⁵ are -H or R⁰.

3. The derivative or pharmaceutically acceptable salt thereof described in claim 1, wherein each of R³, R⁴ and R⁵ is -H.

4. The derivative or pharmaceutically acceptable salt thereof described in claim 3, wherein R⁷ and R⁸ may be the same or different from each other and each represents -H, -R⁰, -OH, -O-R⁰, -O-R⁰⁰-OH or -O-R⁰⁰-O-R⁰, or R⁷ and R⁸ together form oxo group.

5. The derivative or pharmaceutically acceptable salt thereof described in claim 3, wherein R⁷ and R⁸ together form a "lower alkylene which may be interrupted by 1 or 2 divalent groups selected from the class consisting of -O-, -S(O)ₚ-, -NR⁶- and -CONR⁶", and form a 3- to 8-membered ring together with the C atom to which they are linked.

6. The derivative or pharmaceutically acceptable salt thereof described in claim 1, which is selected from the group consisting of N-(diaminomethylene)-9-hydroxy-9H-fluorene-2-carboxamide, 9-chloro-N-(diaminomethylene)-9H-fluorene-2-carboxamide, N-(diaminomethylene)-9-(hydroxyimino)-5-(hydroxymethyl)-9H-fluorene-2-carboxamide, 8-chloro-N-(diaminomethylene)-9-hydroxy-9H-fluorene-2-carboxamide, N- (diaminomethylene) -9-hydroxy-9-methyl-9H-fluorene-2-carboxamide, N-(diaminomethylene)-9-hydroxy-9-methyl-9H-fluorene-2-carboxamide (optically active substance A), N-(diaminomethylene)-9-hydroxy-9-methyl-9H-fluorene-2-carboxamide (optically active substance B), N-(diaminomethylene)spiro[1,3-dithiolane-2,9'-fluorene]-2'-carboxamide, N-(diaminomethylene)-4' 5'-dihydro-3'H-spiro[fluorene-9,2'-furan]-2-carboxamide, N-(diaminomethylene)-4',5'-dihydro-3'H-spiro[fluorene-9,2'-furan]-2-carboxamide (optically active substance A), N-(diaminomethylene)-4',5'-dihydro-3'H-spiro[fluorene-9,2'-furan]-2-carboxamide (optically active substance B), N-(diaminomethylene)spiro[cyclopropane-1,9'-fluorene]-2'-carboxamide, N-(diaminomethylene)-9-methoxy-9-methyl-9H-fluorene-2-carboxamide, N-(diaminomethylene)-9-ethyl-9-methoxy-9H-fluorene-2-carboxamide, N-(diaminomethylene)-5-fluoro-9-hydroxy-9-methyl-9H-fluorene-2-carboxamide, N-(diaminomethylene)-5-fluoro-9-hydroxy-9-methyl-9H-fluorene-2-carboxamide (optically active substance A), N-(diaminomethylene)-5-fluoro-9-hydroxy-9-methyl-9H-fluorene-2-carboxamide (optically active substance B), N-(diaminomethylene)-5'-fluorospiro[1,3-dithiolane-2,9'-fluorene]-2'-carboxamide and N-(diaminomethylene)-5-fluoro-9-methoxy-9-methyl-9H-fluorene-2-carboxamide.

7. A pharmaceutical composition comprising the derivative or pharmaceutically acceptable salt thereof described in claim 1 and a pharmaceutically acceptable carrier.

8. The pharmaceutical composition described in claim 7, which is a 5-HT_{2B} receptor and 5-HT₇ receptor dual antagonist.

9. The pharmaceutical composition described in claim 7, which is a prophylactic antimigraine agent.

10. Use of the derivative or pharmaceutically acceptable salt thereof described in claim 1 for producing a 5-HT_{2B} receptor and 5-HT₇ receptor dual antagonist or a prophylactic antimigraine agent.

11. A method for preventing migraine, which comprises administering a therapeutically effective amount of the fluorene derivative or pharmaceutically acceptable salt thereof described in claim 1 to a patient.
